# EUROPEAN PATENT SPECIFICATION

(11) **EP 1 528 928 B1**
(45) Date of publication and mention of the grant of the patent: **21.07.2010**
(21) Application number: 03717515.5
(22) Date of filing: 28.04.2003
(51) Int. Cl.: A61K 38/02, A61P 35/00

(54) **METHODS AND COMPOSITIONS FOR MODULATING BETA-CATENIN PHOSPHORYLATION**
METHODEN UND ZUSAMMENSETZUNGEN ZUR BEEINFLUSSUNG DER PHOSPHORYLIERUNG DES BETA-CATENINS
PROCEDE ET COMPOSITION POUR MODULER LA PHOSPHORYLATION DE LA BETA-CATENINE

(30) Priority: 29.04.2002 IL 14940402
(43) Date of publication of application: 11.05.2005
(73) Proprietor: YISSUM RESEARCH DEVELOPMENT COMPANY OF THE HEBREW UNIVERSITY OF JERUSALEM, 91390 Jerusalem (IL)
(72) Inventor: BEN-NERIAH, Yinon, 90805 Mevasseret Zion (IL); ALKALAY, Irit, 99875 Zur Hadassah (IL); AMIT, Sharon, 84965 Omer (IL); BIRMAN, Yaara, 23040 Migdal HaEmek (IL); HATZUBAI, Ada, 90870 (IL)
(74) Representative: Weiss, Wolfgang
(86) International application number: PCT/IL2003/000340
(87) International publication number: WO 2003/092719

(56) References cited:
- EP-A- 0 551 200
- EP-A- 1 170 008
- WO-A-00/38709
- WO-A-01/32708
- WO-A-02/24941
- WO-A-94/04541
- WO-A-94/09160
- WO-A-98/57982
- WO-A-99/04238
- WO-A-99/22000

## Description

### Field of the Invention

The present invention relates to the field of cancer treatment. More specifically, the present invention discloses modulators of β-catenin, methods for screening such modulators, as well as compositions and uses thereof.

### Background of the Invention

Genetic studies in flies, frogs and mammals positioned the Wnt pathway as a master regulator in animal development, both during embryogenesis and in the mature organism [Wodarz, A. and R. Nusse (1998) Annu Rev Cell Dev Biol 14 : 59-88; Eastman, Q. and R. Grosschedl (1999) Curr Opin Cell Biol 11: 233-240; Peifer, M. and P. Polakis (2000) Science 287: 1606- 1609; Huelsken, J. and W. Birchmeier (2001) Curr Opin Genet Dev 11: 547-553]. A major target of the Wnt pathway is cytoplasmic-catenin which, when stabilized in response to Wnt signaling, enters the nucleus and serves as a coactivator of TCF/LEF-induced transcription [Willert, K. and R. Nusse (1998) Curr Opin Genet Dev 8: 95-102; Bienz, M. and H. Clevers (2000) Cell 103: 311-320; Polakis, P. (2000) Genes Dev 14: 1837- 1851]. Unstimulated cells harbor a cytoplasmic multiprotein complex containing β-catenin, the Ser/Thr kinase glycogen synthase kinase-3β (GSK-3β), axin [Zeng, L. et al. (1997) Cell 90: 181-192; Ikeda, S. et al. (1998) EMBO J 17: 1371-1384; Sakanaka, C. et al. (1998) Proc Natl Acad Sci U. S. A. 95: 3020-3023], or its homolog Axil/Conductin [Behrens, J. et al. (1998) Science 280: 596-599; Yamamoto, H. et al. (1998) Mol Cell Biol 18: 2867-2875], and the adenomatous polyposis coli (APC) tumor suppressor [Groden, J. et al. (1991) Cell 66: 589-600; Kinzler, K.W. et al, (1991) Science 253: 661-665]. APC and axin are thought to play a scaffold function, facilitating the GSK-3β phosphorylation of β-catenin at the N-terminal region [Hart, M.J., R. et al. (1998) Curr Biol 8: 573-581; Hinoi, T. et al. (2000) J Biol Chem 275: 34399-34406]. This phosphorylation event/s marks β-catenin for ubiquitination by the SCF^{β}-TrCP E3 and subsequent proteasomal degradation [Aberle, H. et al. (1997) EMBO J 16: 3797-3804; Hart, M. et al. (1999) Curr Biol 9: 207-210; Kitagawa, M. et al. (1999) EMBO J 18: 2401-2410; Latres, E. et al. (1999) Oncogene 18: 849-854; Winston, J.T. et al. (1999) Genes Dev 13: 270-283].

Wnt signaling is initiated by secreted Wnt proteins, which bind to members of the frizzled receptor family [Wodarz and Nusse (1998) id *ibid*.]*.* Wnt binding results in the activation of Dishevelled (Dv1-1, 2 and 3 in humans and mice) [Boutros, M. and M. Mlodzik (1999) Mech Dev 83: 27-37], which, via its association with axin, prevents GSK-3β from phosphorylating β-catenin, leading to its stabilization [Yamamoto, H. et al. (1999) J Biol Chem 274: 10681-10684]. The mechanism of Dvl action in inhibiting β-catenin phosphorylation by GSK-3β is mostly unknown. According to a prevailing model it involves PRAT (GBP), a GSK-3β-binding protein that displaces axin from GSK-3β, resulting in failure to phosphorylate β-catenin [Li, L. et al (1999) EMBO J 18: 4233-4240; Farr, G.H. et al. (2000) J Cell Biol 148: 691-702; Bax, B. et al. (2001) Structure (Camb) 9: 1143-1152; Fraser, E. et al. (2002) J Biol Chem 277(3):2176-85]. However, there is currently no evidence linking Dvl activity directly to FRAT-induced axin-GSK-3β dissociation.

The importance of β-catenin phosphorylation in controlling its degradation has been mainly inferred from studies of N-terminal β-catenin mutations in tumor cells [Polakis (2000) *id ibid.*]*.* These, like aberrations of APC or axin, lead to excessive accumulation of β-catenin in the nucleus and deregulated expression of its target genes, promoting neoplastic transformation [Morin, P. J. et al. (1997) Science 275: 1787-1790; Rubinfeld, B. et al. (1997) Science 275 : 1790-1792; Sparks, A. B. et al. (1998) Cancer Res 58: 1130-1134]. β-catenin mutations cluster around the SCF-TrCP binding site and are, therefore, thought to compromise β-catenin ubiquitination and its consequent degradation [Wong, C. M. et al. (2001) Cancer 92: 136-145]. Many of these stabilizing mutations occur at Ser/Thr residues along a putative GSK-phosphorylation site that molds the E3 ubiquitin ligase binding motif, emphasizing the role of GSK-3β in determining β-catenin stability [Polakis (2000) id ibid.]. Yet, Serine **45** (S45), the single most frequent tumor mutation spot, does not conform to a GSK-3β site [Polakis (2000) id ibid.; Wong et al. (2001) id ibid.]. This may have contributed to the notion that β-catenin is an unusual GSK-3β substrate which obviates the need for priming-phosphorylation as a trigger for initiating a GSK-3β phosphorylation cascade [Ding, V. W. et al. (2000) JBiol Chem 275: 32475-32481; Cohen, P. and S. Frame (2001) Nat Rev Mol Cell Biol 2: 769-776; Harwood, A. J. (2001) Cell 105 : 821-824]. Contrary to this view, the inventors' results show that β-catenin phosphorylation at S45 is induced by an axin-CKI complex independently of GSK-3β. Furthermore, this molecular event appears to constitute a major target for Wnt pathway regulation, since the inventors show that Dvl can function as an inhibitor of β-catenin phosphorylation (see Examples).

Hence, based on the inventors' findings, modulators for β-catenin phosphorylation are disclosed. Whereas normally phosphorylated β-catenin is targeted to degradation, in many tumors (particularly in colorectal cancer) the phosphorylation machinery is mutated, the unphosphorylated β-catenin accumulates in the nucleus and functions as a transcriptional activator. Consequently, the disclosed modulators, especially the enhancers of β-catenin phosphorylation are important tools for the prevention and control of the development of cancer, in particular for those types of cancer that result from accumulation and excess activity of non-phosphorylated β-catenin. Surprisingly, certain types of tumors (e. g. , malignant melanoma) accumulate stably phosphorylated β-catenin. In a further disclosure a method to suppress β-catenin phosphorylation in this specific class of tumors, by treating cells with an inhibitor as disclosed is shown.

The invention describes both enhancers and inhibitors of β-catenin phosphorylation. Thus, in addition, a method for screening for modulators, i. e. inhibitors and enhancers of β-catenin phosphorylation is disclosed.

Objects of the invention will become apparent as the description proceeds.

### Summary of the Invention

The present invention relates to modulators of β-catenin phosphorylation and to their use in cancer treatment.

Thus, in a first aspect, the present invention claims a method of screening for an anti-cancer agent, wherein said method comprises the steps of:
(a) contacting an agent with a reaction mixture comprising β-catenin, axin and/or casein kinase I alpha (CKla),
(b) incubating said mixture under conditions which allow a change in phosphorylation of β-catenin at serine 45 (S45), and
(c) detecting phosphorylation of β-catenin at S45,
   whereby enhanced phosphorylation of β-catenin at S45 indicates that said agent is useful as an anti-cancer agent.

As disclosed, a modulator is an inhibitor of β-catenin Serine 45 (S45) phosphorylation, said inhibitor is selected from any one of the proteins Dishevelled (Dvl), a Wnt protein, phosphatase PP2A, and CKI inhibitors. Further disclosed is a modulator, which is an enhancer of β-catenin Serine 45 (S45) phosphorylation, said enhancer is selected from any one of phosphatase inhibitors, acetylases and proteins that promote β-catenin acetylation. Preferably, said phosphatase inhibitor is okadaic acid, and said protein that promotes β-catenin acetylation is FRAT.

The present disclosure shows a method of screening for an agent which modulates β-catenin S45 phosphorylation, wherein said method comprises the steps of :
a. providing a candidate agent, contacting said agent with a reaction mixture comprising β-catenin and any other reagent (s) necessary for β-catenin phosphorylation, wherein said mixture is a cell mixture or a cell-free mixture,
b. incubating said mixture under suitable conditions, and
c. detecting by suitable means whether or not β-catenin S45 has been phosphorylated, wherein said suitable means of detection may be any one of a reaction with a specific antibody, phosphopeptide mapping or mass spectrometry,
   whereby enhanced phosphorylation of β-catenin S45 indicates that said substance is an enhancer of β-catenin phosphorylation, and reduced phosphorylation of β-catenin S45 indicates that said substance is an inhibitor of β-catenin phosphorylation.

Further disclosed is a modulator of β-catenin Serine 45 (S45) phosphorylation which has been identified by the method of screening described in the invention. Said modulator may be an inhibitor or an enhancer of β-catenin S45 phosphorylation.

The present disclosure shows a method of enhancing the phosphorylation of β-catenin in a cell by treating the cell with an enhancer as defined by the invention.

In a further aspect, the present invention relates to a pharmaceutical composition, preferably for the treatment of cancer, comprising CKI-7.

In yet a further embodiment, the present invention relates to the use of CKI-7 for the manufacture of a medicament for the treatment of cancer, preferably wherein β-catenin of said cancer is stabilized and its phosphorylation is not impaired.

In a yet further embodiment, the present invention comprises the use of CKI-7 for the treatment of cancer, particularly malignant melanoma.

Further dislcosed is the use of the modulator of β-catenin phosphorylation for the treatment of cancerous cells wherein β-catenin is stabilized and its phosphorylation is impaired, as in colon adenoma or carcinoma, for example, and said modulator is an enhancer of β-catenin phosphorylation. Alternatively, the use of the modulator of β-catenin phosphorylation, as defined by the invention, is for the treatment of cancerous cells wherein β-catenin is stabilized and its phosphorylation is not impaired, as in melanoma, for example, and said modulator is an inhibitor of β-catenin phosphorylation In a second embodiment, the modulator of β-catenin phosphorylation, as defined by the invention, is to be used in the preparation of a pharmaceutical composition for the treatment of cancer, i.e. CKI-7.

In a further disclosure, the modulator of β-catenin phosphorylation is to be used in the preparation of a parmaceutical composition for the treatment of cancer.

Further objects of the present invention are the use of a siRNA of CKI-α for the manufacture of a medicament identified for the treatment of cancer and a pharmaceutical composition comprising a siRNA of CKI-α.

In a further disclosure, the modulator of β-catenin phosphorylation is to be used in the preparation of a pharmaceutical composition for the treatment of cancerous cells. It should be noted that in those types of cancer where phosphorylation of catenin is impaired, the modulator of choice shall be an enhancer of catenin phosphorylation. Alternatively, in those types of cancer where phosphorylation of β-catenin is not impaired, the modulator of choice shall be an inhibitor of β-catenin phosphorylation.

### Brief Description of the Figures

**Figure 1 a-c** : Axin induces β-catenin phosphorylation exclusively on S45, yet proteasomal degradation requires further GSK-mediated phosphorylation at T41, S37 and S33.
Fig. 1a: β-catenin [wt or N-terminal-mutated β-catenin (DP)] stability in response to transfection of axin and GSK-3β. A dominant negative Flag-ΔF-box-β-TrCP fragment (WD) was included in lane 7. A GFP expression vector was included in all transfections as an expression reference.
Fig. 1b: β-catenin phosphorylation analysis with anti-phosphopeptide antibodies in proteasome-inhibited cells.

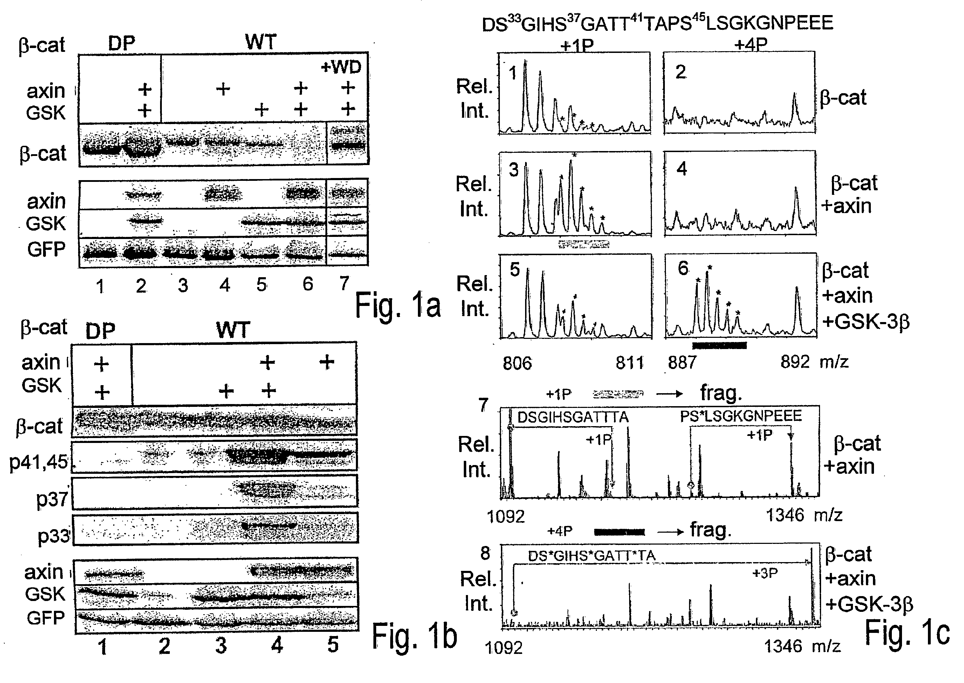

Fig. 1c: β-catenin phosphorylation analysis by mass spectrometry (MS). Immunopurified Flag-β-catenin bands were digested with Asp-N endoproteinase and the resulting N-terminal peptides, including Aspl7-Leu31 and Asp32-Glu55 (showed on top) were analyzed by nanoelectrospray MS. The m/z ratios shown (6 top panels) correspond to triple-charged [M+3H] 3+ ions of the Asp32-Glu55 peptide, and is marked by asterisks (including all its isotopic variants). Displayed are spectra of peptides containing 1 (+1P) and 4 (+4P) phosphate groups from p-catenin alone (panels 1,2), β-catenin co-expressed with axin (panels 3,4), and axin plus GSK-3β (panels 5, 6). [M+3H] 3+ ions from bar-marked peptides (grey and black) were selected for fragmentation. The spectra in the lower panels (7, 8), correspond to fragments derived from MS/MS of the grey bar peptide (from panel 3) and black bar peptide (from panel 6). The Asp32-Glu55 peptide was fragmented by MS/MS into halves, a C-terminal half containing Ser45 and an N-terminal half containing Ser33/Ser37/Thr41. The grey bar C-terminal fragment is mostly phosphorylated, whereas the N-terminal fragment is mostly unphosphorylated (panel 7). The relationship of non-phosphorylated to phosphorylated (+1P) fragments is indicated by angled arrows. The black bar N-terminal fragment (panel 8) is mostly tripli-phosphorylated (+3P). Its C-terminal fragment is singly phosphorylated (not shown), similarly to that of the grey bar. MS/MS analysis of the Asp17-Leu 31 peptide (from +1P of the MS), reveals minor, yet significant Ser29 phosphorylation (not shown).

Abbreviations: frag., fragments.
**Figure 2a****-b**: Mutations at four β-catenin N-terminal phosphorylation sites curtails the phosphorylation cascade, leading to β-catenin stabilization.

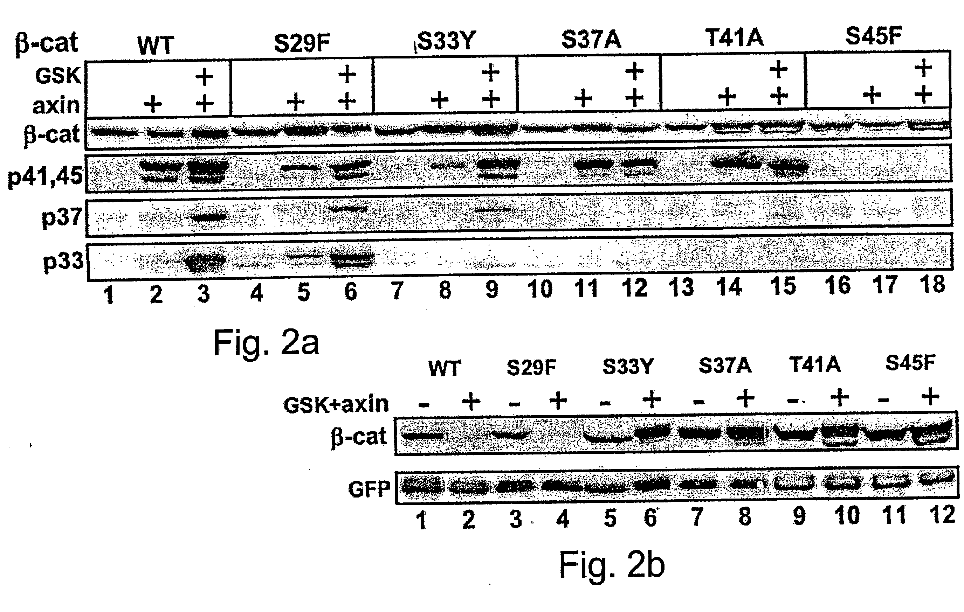

Fig. 2a: Wild-type (wt) and mutated β-catenin from MG-132 treated cells was detected with anti-Myc or anti-phospho-β-catenin antibodies.
Fig. 2b: Stability analysis of phosphorylation=site β-catenin mutants in MG-132 untreated cells.

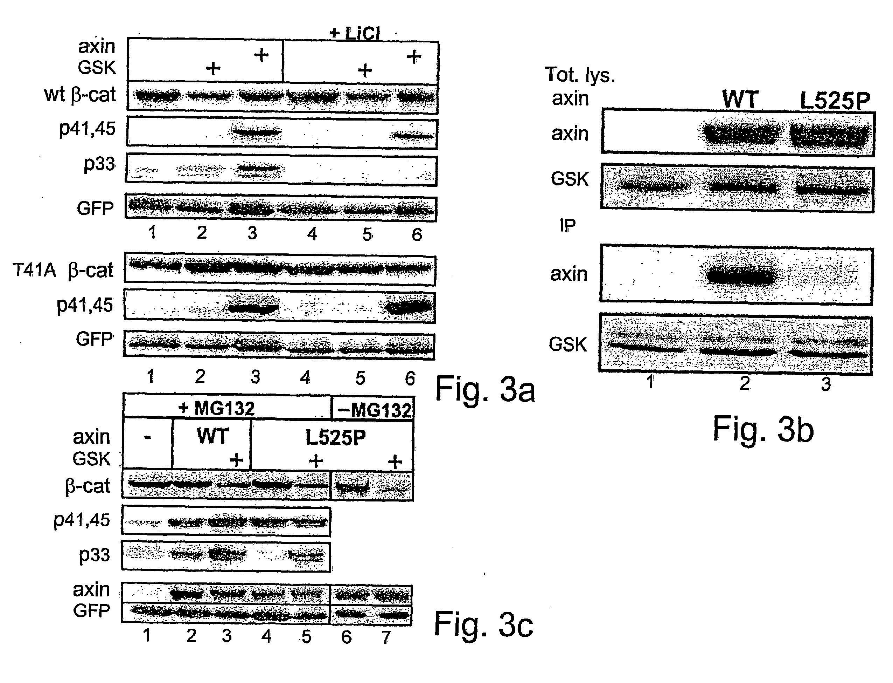

**Figure 3a****-c**: Axin-mediated S45 phosphorylation is GSK-3β-independent.
Fig. 3a: S45 phosphorylation is not affected by LiCl. Analysis of wt, or T41A β-catenin from MG-132-treated cells, with or without LiCl. No phosphorylation signal at 833/87 was detected in T41A mutant (see Fig. 2a):
Fig.3b: L525P-axin does not bind GSK3β. Anti-Flag immunoprecipitation from cells transfected with Flag-GSK3β and wt or L525P Myc-axin.
Fig. 3c: L525P-axin induces S45 phosphorylation and supports a phosphorylation-degradation cascade in the presence of exogenous GSK-3β. All cells were transfected with Myc-β-catenin and the indicated expression vectors.

Abbreviations: Tot. lys., total lysate.

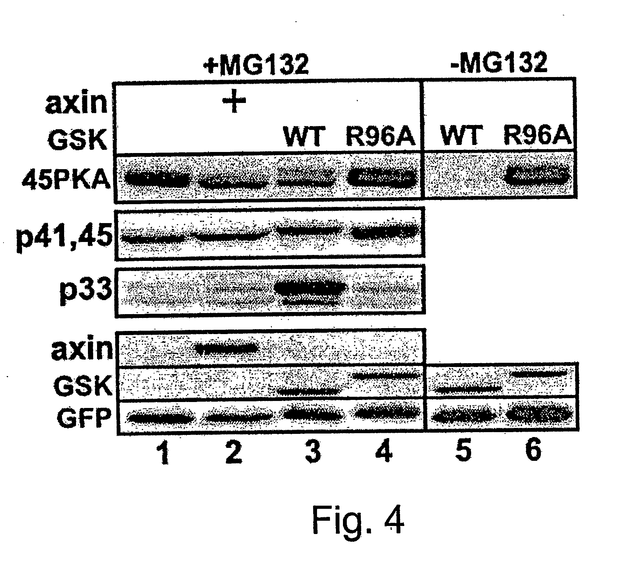

**Figure 4**: Axin-independent, surrogate phosphorylation at S45 promotes the GSK-3p phosphorylation cascade, resulting in β-catenin degradation. All cells were transfected with the β-catenin mutant 45PKA and the indicated expression vectors. HA-GSK-3β (W1) or Flag-R96A-GSK-3β (R96A) were detected using anti-GSK-3β antibodies (GSK-3β position varies according to its tag).

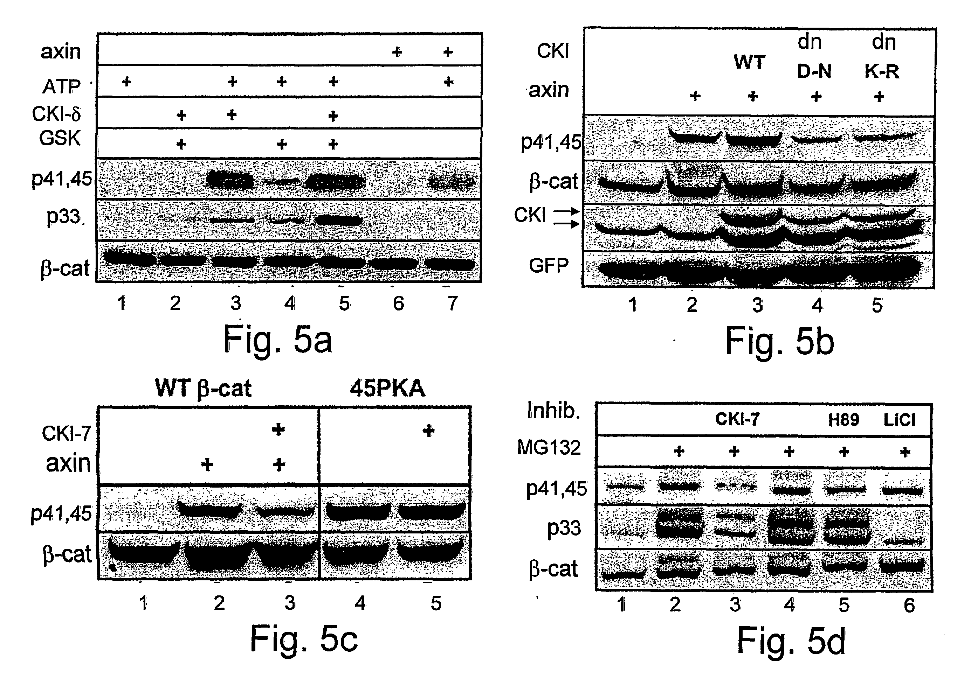

**Figure 5a****-e**: Identification of the β-catemn S45 kinase.
Fig.5a: Immunopurified axin and recombinant CKIδ (aa1-318) phosphorylate β-catenin *in vitro* at S45, creating a priming site for sequential phosphorylation at S33/37 by recombinant GSK-3β. Flag-β catenin was immunoprecipitated from 293 transfected cells and subjected to an *in vitro* kinase assay using the indicated kinase or immunopurified Flag-axin from 293 transfectants, with or without ATP (30µM) and analyzed by Western blot, using anti-phospho-β-catenin antibodies.
Fig. 5b: Dominant negative CKI inhibits axin-induced S45 phosphorylation. 293 cells were transfected with Myc-β-catenin alone (lane 1), with axin (lanes 2-5) and wt- (lane 3), or dn-CKIε (D-N, lane 4; K-R, lane 5). The bottom CKI arrow points to the endogenous hCKIε, whereas the top arrow points to the exogenous xCKIε proteins detected by monoclonal anti-CKIε.
Fig. 5c: CKI-7 inhibits the axin-induced S45 phosphorylation of wt ε-catenin, but not the constitutive phosphorylation of 45PKA.
Fig. 5d: Effects of specific kinase inhibitors on the phosphorylation of endogenous β-catenin at S41,45 and S33 in HeLa cells. CKI-7 was used at 100µM, H89 at 5µM and LiCl at 40mM. β-catenin was detected with anti-β-catenin antibody, or anti-phospho-β-catenin antibodies.

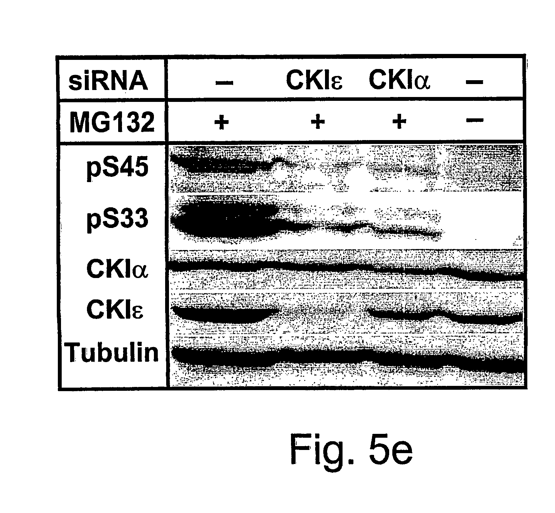

Fig. 5e: Comparable contribution of CIK alpha and epsilon to β-catenin phosphorylation in RKO cells.

Abbreviations: inhib., inhibitor.

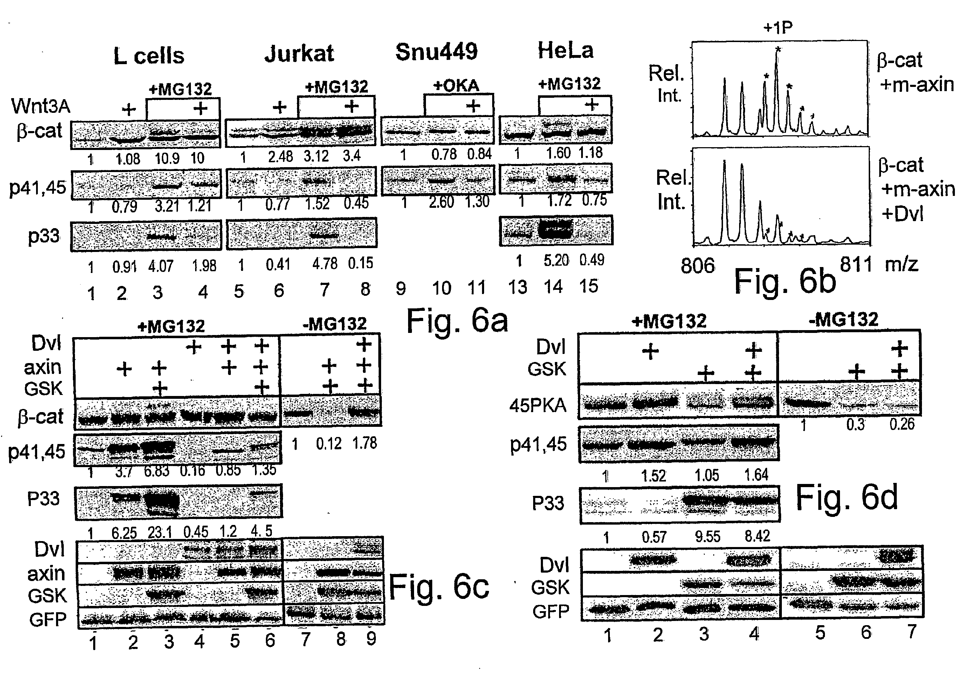

**Figure 6a****-d**: The Wnt pathway targets the axin-mediated priming phosphorylation of β-catenin at S45.
Fig. 6a: Wnt3A inhibits S45 phosphorylation. Mouse L cells, SNU449 hepatoma cells [Satoh et al.(2000) Nat Genet 24(3):245-50], HeLa cells and β-catenin transfected Jurkat T cells were incubated (5 hr) with conditioned medium of wt or Wnt3A secreting L cells [Shibamoto et al. (1998) Genes Cells 3: 659-670]. MG-132 or okadaic acid (OKA, 1µM were added to the indicated cultures. Endogenous (HeLa, L cells and SNU449) and exogenous (Jurkat) β-catenin was detected with anti-β-catenin antibody, or anti-phospho-β-catenin antibodies. The numbers under the different lanes are relative densitometry figures, preferring to the signal intensity of untreated cultures.
Fig. 6b: Dv1-1 inhibits axin-mediated S45 phosphorylation. Mass spectra of the Asp32-Glu55 peptide with one phosphate group (+1P): from β-catenin co-expressed with L525P-axin (m-axin), compared with that of β-catenin co-expressed with L525P-axin and Dv1-1.
F5.g.6c: Dv1-1 overexpression results in inhibition, of S45 phosphorylation and β-catenin stabilization. β-catenin of 293 transfectants was detected using anti-Myc or anti-phospho-β-catenin antibodies.
Fig.6d: Dvl-1 has no effect on axin-independent, S45-phosphorylation-initiated GSK3β cascade: Analysis of 45PKA-transfected cells.

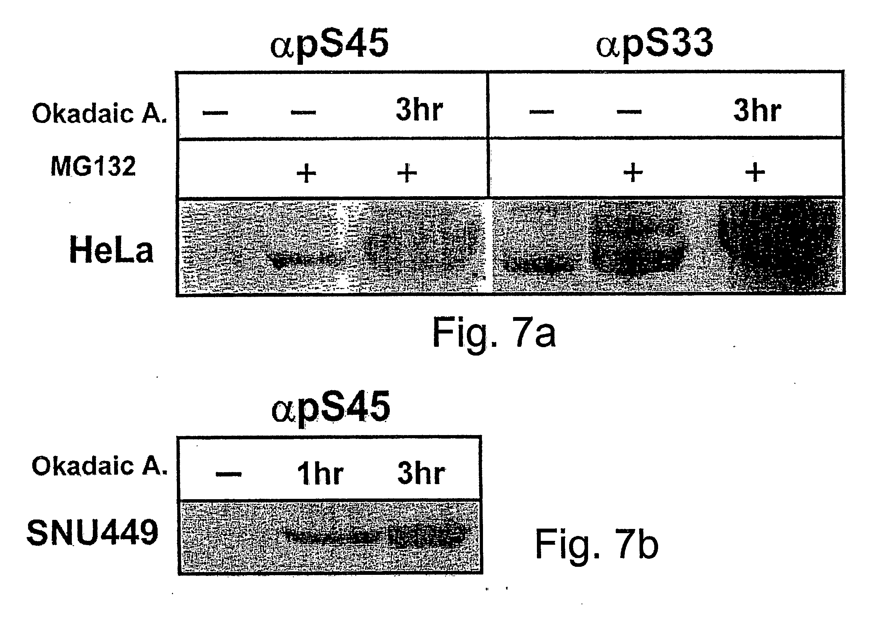

**Figure 7a****-c**: Okadaic Acid treatment induces Ser45 and Ser33 phosphorylation and S45 kinase activity.
Fig. 7a: HeLa cells were incubated with MG132 with or without okadaic acid (1µM for 3hr). Cell lysates were analyzed by Western blotting with αp45 and αp33.
Fig. 7b: SNU449 cells [Satoh *et al*. (2000) *id ibid*.] were treated with okadaic acid (1 µM for 1 or 3 hr), and their lysates tested for S45 phosphorylation by Western blotting,

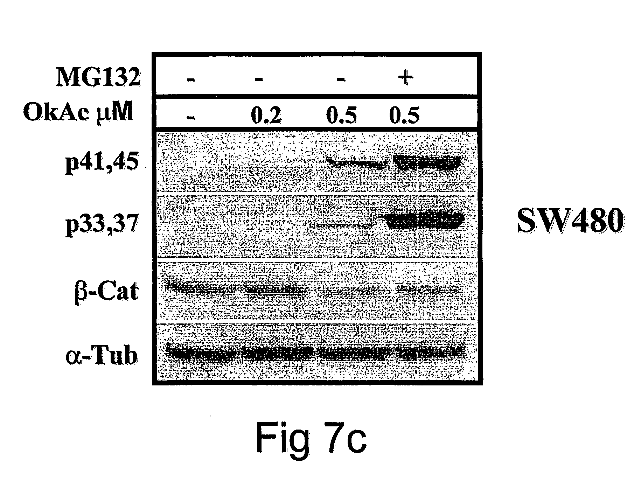

Fig.7c: Okadaic Acid induces β-Catenin phosphorylation and degradation in the APC mutated cell line SW480.

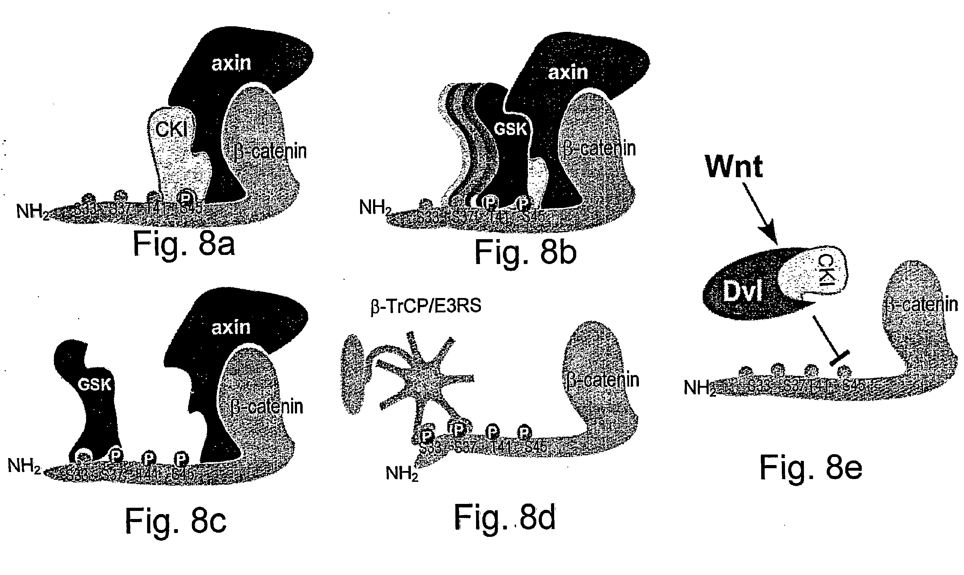

**Figure 8a****-e**: Putative model depicting the β-catenin phosphorylation/degradation cascade.
Fig. 8a: Axin recruits CKI to phosphorylate β-catenin at S45.
Fig. 8b: S45 phosphorylation primes β-catenin for the succeeding GSK-3β phosphorylation cascade, finally hitting the S33/37 sites.
Fig. 8c: Same as 8b.
Fig. 8d: Phosphorylation at S33/37 creates a docking site for the β-TrCP/E3RS promoting the ubiquitination and degradation of β-catenin.
Fig. 8e: Wnt signaling, possibly through Dvl and CKI, regulates S45 phosphorylation.

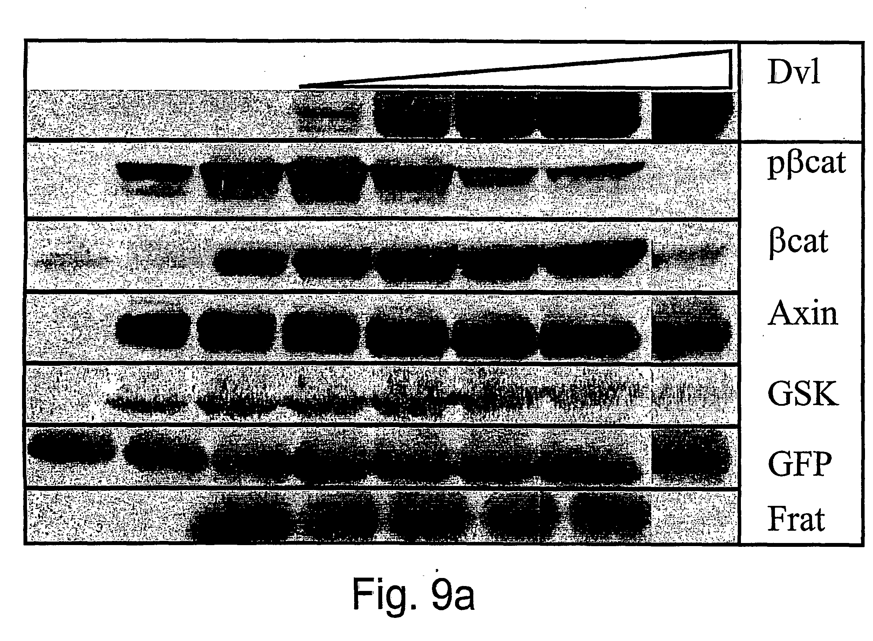

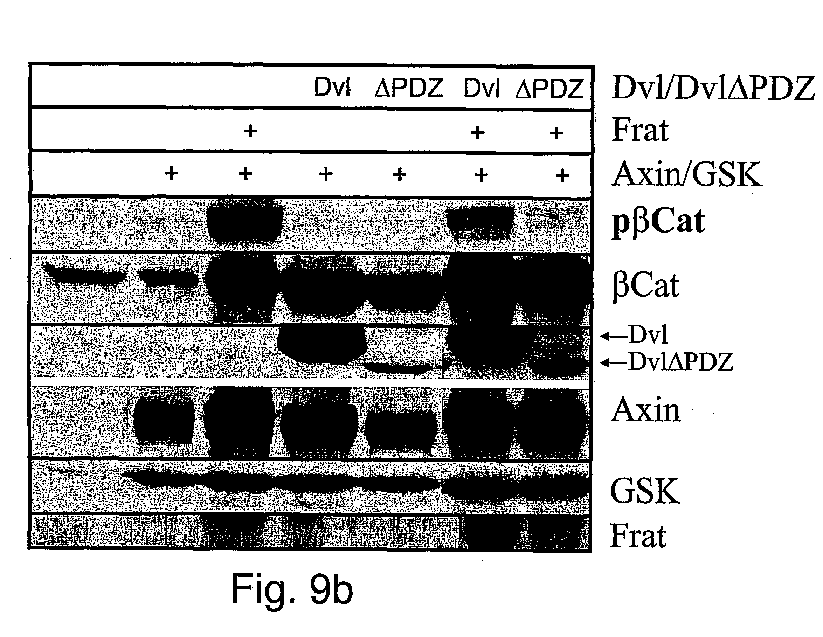

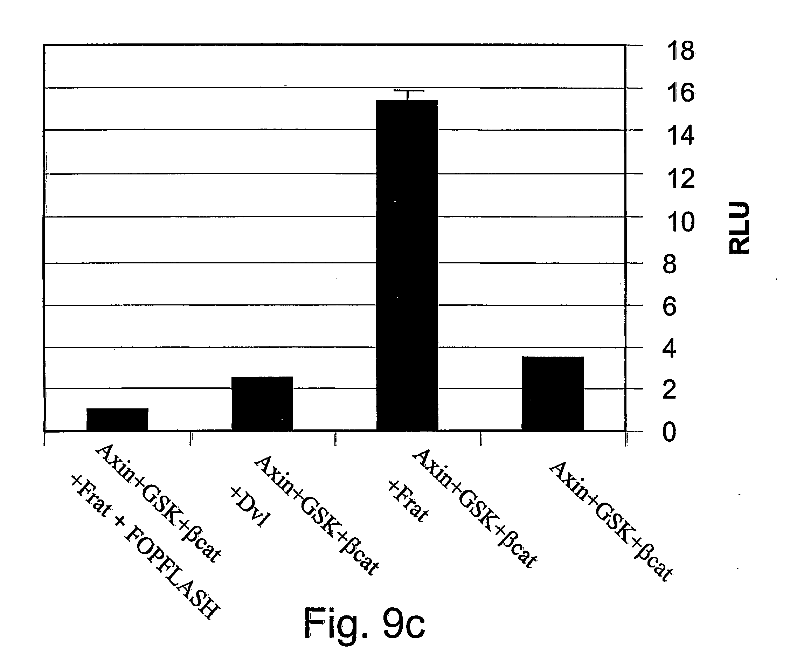

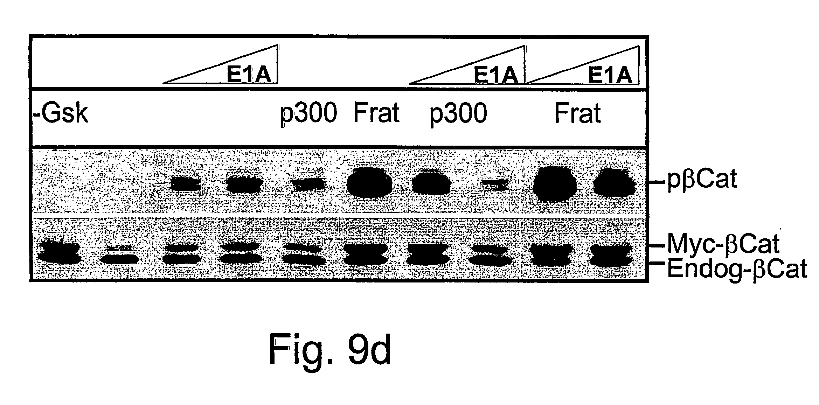

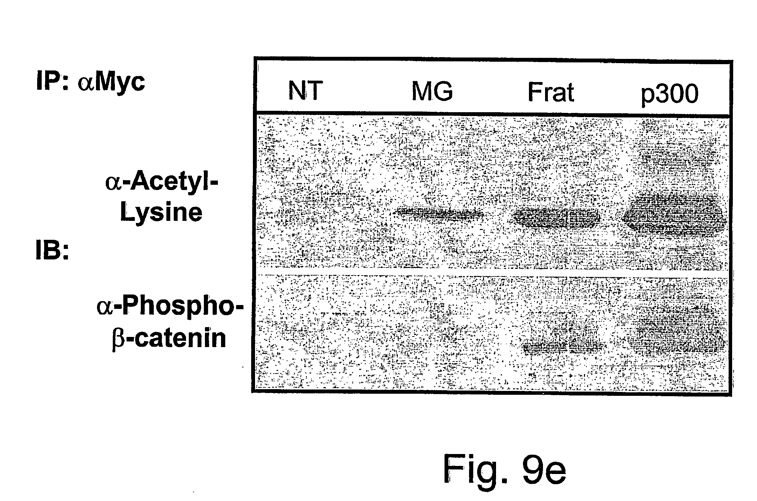

**Figure 9** FRAT1 stabilizes phosphorylated β-catenin
Fig. 9a: Western blot showing the levels of phospho-β-catenin (pβCat), β-catenin (βCat) and the regulatory proteins, as indicated, in transfected 293 cells. Frat1 stabilizes phospho-β-catenin, which is further augmented by low Dvl expression. Dvl expression was varied by a twofold consecutive increase of the transfected plasmid (0.25-4 µg).
Fig. 9b: Western blot showing the expression levels of phospho-β-catenin (pβCat), β-catenin (βCat) and the indicated regulatory proteins. DvlΔPDZ is incapable of cooperating with Frat1 in the stabilization of pliospho-β-catenin, yet suppresses β-catenin phosphorylation on S45, thus inducing the stabilization of non-phosphorylated β-catenin.
Fig. 9c: Frat1 stabilizes a transcriptionally-active phospho-β-catenin in 293 cells. Graph showing the transcriptional activity of phosphorylated β-catenin, through its activation of the luciferase reporter TOPFLASH [Korinek et al. (1997) Science, 275: 1752-3], as expressed in Relative Luciferase Units (RLU), in the presence or absence of Frat1. The TCF mutant-site reporter (FOPFLASH) was used to control the TCF-independent effect of Frat1/axin/GSK transfection.
Fig. 9d: Western blot showing the levels of endogenous and transfected β-catenin, as well as phosphorylated β-catenin, in the presence of p300/CBP, E1A and Frat. E1A expression varied by a twofold consecutive increase of the transfected plasmid (3-6 µg). p300/CBP and E1A stabilizes phosphor-β-catenin similarly to Frat1. The left lane was transfected without GSK3.
Fig. 9e: The phospho-β-catenin stabilized by p300/CBP and Frat1 is acetylated on lysine residues. 293 cells were transfected with Myc-β-catenin and analyzed by immunoprecipitation of Myc-β-catenin and Western blotting, using anti-acetyl-lysine or anti- p33, 37, 41 antibodies (Cell Signaling Inc). MG=treated with MG132-a proteasome inhibitor. NT=without any additional transfected plasmid (except of β-catenin, GSK3 and Axin).

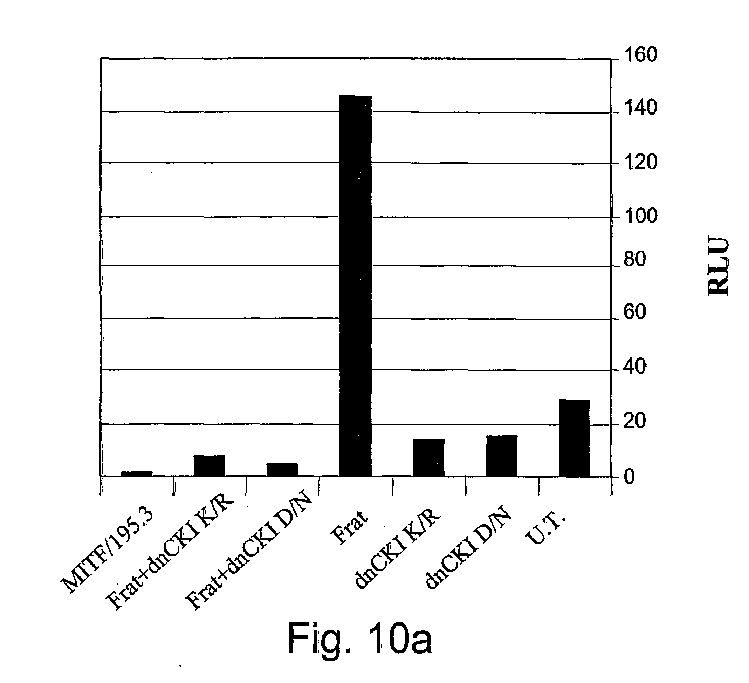

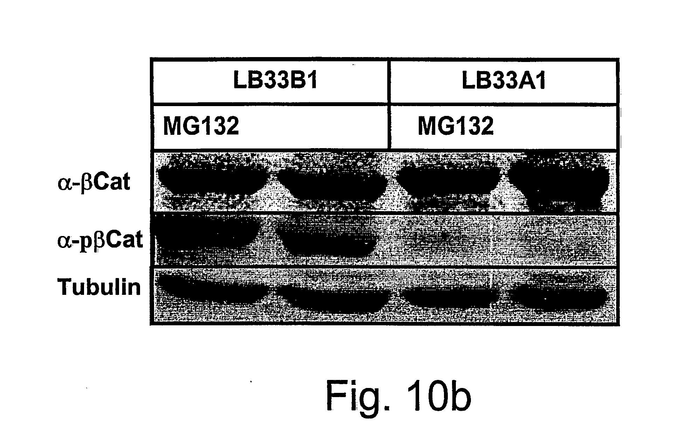

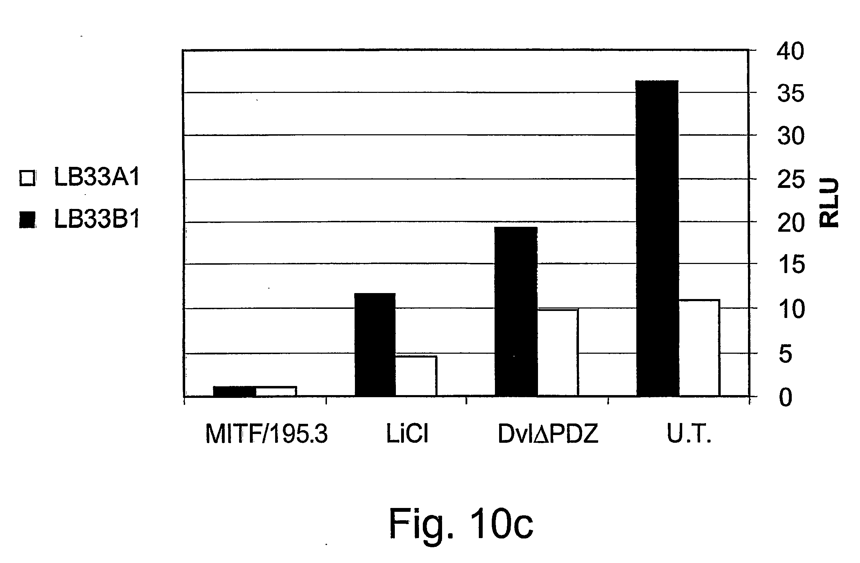

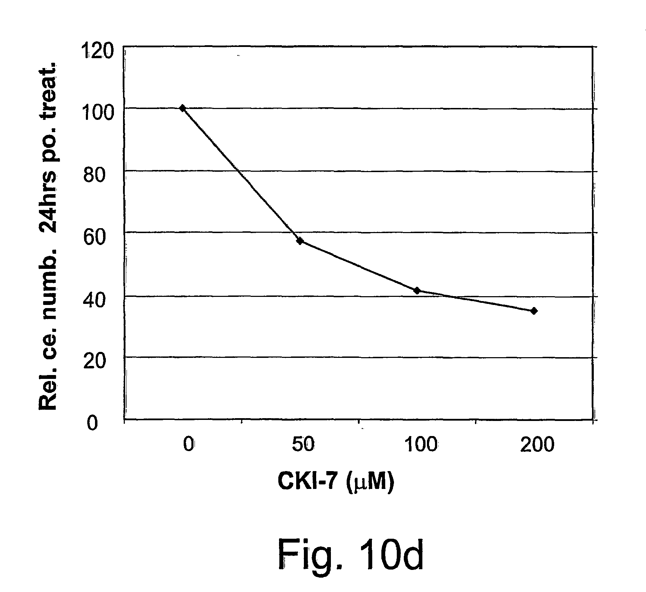

**Figure 10**: β-catenin phosphorylation is essential for the transcriptional activation of MITF-M, a melanoma survival gene and for melanoma growth in tissue culture.
Fig. 10a: Graph showing β-catenin transcriptional activity on the pGL3-MITF/M luciferase reporter plasmid. Frat1-induced transcriptional activity requires β-catenin phosphorylation. The MITF-M transcriptional activity is expressed in Relative Luciferase Units (RLU). A TCF mutant-site reporter (pGL3-MITF/M195.3) was used to control the TCF-independent effect of Frat1 transfection). The dominant negative CKI plasmids K/R and D/N are explained in Fig. 5b.
Fig. 10b: β-catenin phosphorylation is exclusive to the B1 metastatic derivative of the A1 melanoma tumor. LB33A1 and LB33B1 cells were treated with or without 20µM MG132 for 4 hours, harvested and their lysates analyzed by Western blotting with the indicated antibodies.
Fig. 10c: MITF transcriptional activity requires β-catenin phosphorylation. The two related melanoma cell lines LB33A1 and LB33B1 were transfected with the pGL3-MITF/M luciferase reporter plasmid [Takeda et al. (2000) J Biol Chem 275: 14013-6] alone or together with DvlΔPDZ expression plasmid. 24 hours after transfection, one sample of cells was treated with 20mM LiCl for 24 hours, as indicated. Cells were harvested and their lysate luciferase activity was determined using Promega's Luciferase Assay System. MITF-M transcriptional activity is expressed in Relative Luciferase Units (RLU).
A TCF mutant-site reporter (pGL3-MITF/M195.3) was used to control TCF-independent transcription.
   Fig. 10d: Blocking β-catenin phosphorylation in a melanoma cell line affects cell growth. LB33B1 cells were treated with increasing concentrations of the S45 phosphorylation inhibitor CKI7 and live cells were counted 24 hours post treatment.

Abbreviations: U.T., untreated or untransfected; Real. ce. numb. 24 hrs. po. treat., relative cell numbers 24 hours post-treatment.

### Detailed Description of the Invention

The following abbreviations are used throughout this application:
- APC: adenomatous polyposis coli
- CKI: casein kinase I
- DP: dominant positive
- Dvl : Dishevelled
- GFP : green fluorescence protein
- GSK3β (GSK) : glycogen synthase kinase 3β
- MS : mass spectrometry
- OKA : Okadaic acid
- PKA : protein kinase A
- S45 : β-catenin serine 45

The inventors' studies were aimed to address certain key questions in the regulation of the Wnt-β-catenin pathway, namely, what molecular event/s triggers the phosphorylation-degradation cascade of β-catenin and which of them is a target for Wnt regulation.

The present disclosure shows the modulation of β-catenin phosphorylation. Specifically, it comprises modulators of β-catenin serine 45 (S45) phosphorylation, and methods for screening such modulators, as well as compositions and uses thereof.

In one aspect, the present invention comprises the use of a modulator of β-catenin serine 45 (S45) phosphorylation, wherein said modulator is an inhibitor which is CKI-7.

It is to be understood that the term modulator is used throughout this specification as any substance, be it a drug, a compound, a protein or a peptide, capable of enhancing or diminishing β-catenin phosphorylation. The modulator is able to interact with β-catenin directly or indirectly, in such a way that it may enhance or inhibit its phosphorylation. Alternatively, the modulator may affect, i. e., induce or repress the S45 phosphorylation machinery (or the S45 kinase activity).

In addition, another effect of the modulator may be to trigger a change in the intracellular localization of β-catenin, and in that way affect its phosphorylation. Normally, β-catenin is found in two intracellular pools: bound to E-cadherin at the cell membrane, and in a complex with other proteins, including APC and axin, in the cytoplasm. Upon activation, β-catenin is translocated to the nucleus. Phosphorylation or de-phosphorylation of β-catenin might thus affect its intracellular localization and cause its translocation between these different cellular compartments.

Said inhibitor, as disclosed, is selected from any one of the Dishevelled (Dvl) protein, a Wnt protein, and the phosphatase PP2A.

Contrary to the common knowledge in the field, the inventors' experiments surprisingly indicated that the axin/CKI-mediated phosphorylation of β-catenin at S45, rather than GSK3-mediated β-catenin phosphorylation, is the major regulated molecular event of the Wnt signaling pathway. Liu et al. published results pointing to a dual-kinase mechanism for β-catenin phosphorylation-degradation [Liu, C. et al. (2002) Cell 108: 837-847]. However, Liu's results conform to the prevailing notion that Wnt signaling regulates β-catenin stabilization through GSK3. This is in sharp contrast to the results presented here, wherein Wnt3A signaling and Dvl overexpression regulate S45 phosphorylation independently of GSK3. To observe the full extent of S45 phosphorylation, β-catenin has to be stabilized. This may be achieved by proteasomal inhibition, or by using a cell line mutated at phosphorylation sites upstream of S45 (see Fig. 6a). Under these conditions, all four tested cell lines (including a colon carcinoma-derived cell line harboring a S37 mutated β-catenin) responded to Wnt3A by downregulating the entire phosphorylation cascade from S45 onwards (Fig. 6a). Thus, the inventors' results show that S45 phosphorylation is the primary step for β-catenin phosphorylation cascade, and it is regulated by Wnt signaling via axin and independent of GSK3β.

As demonstrated in Example 7 and Fig. 10d, abrogation of β-catenin phosphorylation with CKI7, a CKI inhibitor, in LB33B1 melanoma cells affected their proliferation.

Hyper-phosphorylated β-catenin is often detected in certain types of tumors, such as human malignant melanomas, where it accumulates specially in the nucleus [Kielhorn, E. et al. (2003) Int. J. Cancer 103 (5): 652-6]. It appears that stabilized phosphorylated p-catenin contributes to the induction of a unique set of TCF target genes, distinct from the known TCF-regulated genes (which are targets of non-phosphorylated β-catenin). One example of this different set of genes might be MITF-M, a transcription factor that is necessary for the survival of melanoma cells [Widlund, H. R. et al. (2002) J. Cell Biol. 158 (6): 1079-87]. Hence, phosphorylated β-catenin may also play an important role in the tumorigenesis process, possibly as a transcriptional activator of potential oncogenes. Inhibiting β-catenin phosphorylation at S45 would thus be the means of repressing critical tumorigenesis factors, such as MITF-M.

PP2A was implicated before in Wnt signaling, but its target site and effect (i. e. , S45) were unknown. By inhibiting the PP2A phosphatase, okadaic acid or a similar agent can be useful in inducing the axin-CKI phosphorylation activity, resulting in enhanced β-catenin degradation in APC-mutated colon cancer and possibly in other tumors. This is because overexpression of axin in APC-or axin-mutated cells can drive β-catenin degradation [Kishida S. et al. (1998) J Biol Chem 273 (18): 10823-6] and tumor apoptosis [Satoh S. et al. (2000) id ibid.]. By augmenting the activity of endogenous axin, okadaic acid would induce the apoptosis of the tumor. Therefore, compounds that facilitate S45 phosphorylation will be useful as anti-cancer agents.

It is further disclosed that said enhancer of β-catenin phosphorylation is any one of FEAT, p300/CBP, E1A, or any other protein-acetylase. The enhancer of β-catenin phosphorylation may be any protein, or factor, as for example a histone deacetylase (HDAC) inhibitor, that promotes β-catenin acetylation. Preferably, said enhancer is FRAT, including any of its isoforms FRAT1, FRAT2 or FRAT3 [Chai, J. H. et al. (2001) Mamm. Genome 12 (11): 813-21; Saitoh and Katoh (2001) Int. J. Oncol. 19 (2): 311-5; Jonkers, J. et al. (1997) EMBO J. 16 (3): 441-50].

In Example 6, the inventors showed that the oncoprotein Fratl [Jonkers, J. et al. (1997) id ibid.], commonly thought to function as a GSK3 phosphorylation inhibitor [Li, L. et al. (1999) EMBO J. 18 (15): 4233-40; Salic, A. et al. (2000) Mol. Cell 5 (3): 523-32], is in fact an enhancer of β-catenin phosphorylation. In addition, as shown in Figure 9d, the inventors identified two other enhancers of β-catenin phosphorylation, adenovirus EIA and p300/CBP [Goodman and Smolik (2000) Genes Dev. 14 (13): 1553-77], which when overexpressed in 293 cells induce the accumulation of hyper-phosphorylated β-catenin. The inventors' results suggest that phosphorylated β-catenin undergoes acetylation of its Lys residues. This phosphorylated and acetylated β-catenin is resistant to degradation, and can function as a transcriptional activator.

The present disclosure further shows a method of screening for an agent which modulates β-catenin S45 phosphorylation, wherein said method comprises the steps of :
a. providing a candidate agent, contacting said agent with a reaction mixture comprising β-catenin and any other reagent(s) necessary for β-catenin phosphorylation, wherein said mixture is a cell mixture or a cell-free mixture,
b. incubating said mixture under suitable conditions, and
c. detecting by suitable means whether or not β-catenin S45 has been phosphorylated,
   whereby enhanced phosphorylation of β-catenin S45 indicates that said substance is an enhancer of β-catenin phosphorylation, and reduced phosphorylation of β-catenin S45 indicates that said substance is an inhibitor of β-catenin phosphorylation.

The method may be carried out in a cell mixture or a cell-free mixture, comprising β-catenin, axin, optionally CKI and GSK3β, and any other reagent necessary for β-catenin phosphorylation. β-catenin S45 phosphorylation may be observed by any means suitable for detection of protein phosphorylation. Preferably, β-catenin S45 phosphorylation is detected by a specific anti-phosphopeptide antibody, by phosphopeptide mapping or mass spectrometry.

As demonstrated in the Examples, TCF-regulated genes might be used as an end-point for the method of the invention. Thus, enhancers of ss- catenin phosphorylation shall induce the expression of a TCF reporter upon transfection of said reporter in combination with the enhancer being tested, in the cells of interest. Another reporter gene to be used as an end- point for the method of the invention is the MITF reporter plasmid.

Enhancers of β-catenin phosphorylation shall induce the expression of a MITF reporter plasmid, whereas inhibitors of β-catenin phosphorylation shall suppress its expression.

Another end-point to be used in the method of the invention is the rate of proliferation of certain cell lines in the presence of phosphorylated or non-phosphorylated β-catenin. As shown in Example 7, B1 melanoma cells slowed their proliferation in the presence of the specific CKI inhibitor. Thus, factors that are able to slow the growth of B1 melanoma cells, for example, shall be identified as inhibitors of β-catenin phosphorylation.

As demonstrated in Examples 1, 2 and 3, a major role of axin in the Wnt pathway is to provide the kinase activity that initiates the β-catenin phosphorylation cascade at S45. This process is mediated by CKI isoforms α, β or ε, since all were detected in association with axin by LC/MS. Yet, under specific physiological settings, a particular CKI isoform might function with axin. Association of axin with a single CKI isoform may require an intermediate molecule. S45 phosphorylation by the axin-CKI complex is necessary and sufficient to mobilize a GSK3-mediated cascade.

Therefore, in the method of screening for a modulator of β-catenin, the reaction mixture may preferably contain axin and/or CKI. Specifically, the axin may be immunopurified from transfected cells.

Without being bound by theory, a model for the β-catenin phosphorylation/degradation cascade may be proposed, as depicted in Fig. 8. In this model, axin binds to β-catenin and recruits CKI to phosphorylate S45. S45 phosphorylation primes β-catenin for the succeeding GSK-3β phosphorylation cascade, finally hitting the S33/37 sites. Phosphorylation at S33/37 creates a docking site for the β-TrCP/E3RS complex, which promotes the ubiquitination and degradation of β-catenin. Wnt signaling, possibly through Dvl and CKI, regulates S45 phosphorylation, by eliminating axin (e. g., by inducing its degradation), or quenching the recruitment of CKI to S45. Alternatively, Wnt signaling, possibly through PP2A, induces the dephosphorylation of β-catenin at S45.

Hence, the disclosure shows a modulator of β-catenin Serine 45 (S45) phosphorylation which is identified by the method of screening described in the invention. Said modulator may be an inhibitor or an enhancer of β-catenin S45 phosphorylation.

The modulator identified by the method of the invention can be used to enhance or to inhibit β-catenin phosphorylation, preferably in β-catenin S45 residue.

Further disclosed is a method of enhancing the phosphorylation of β-catenin in a cell, by treating the cell with a modulator of β-catenin phosphorylation, wherein said modulator is an enhancer of β-catenin phosphorylation, such as a phosphatase inhibitor. Said enhancer may be okadaic acid. Alternatively, said enhancer may be a protein that promotes β-catenin acetylation.

As demonstrated in Example 6, proteins like E1A and FRAT1 can induce the stabilization of phosphorylated β-catenin, likely by protein acetylation. Thus, FRAT1 is another preferred enhancer of β-catenin phosphorylation.

In a further aspect the present invention provides a pharmaceutical composition for the treatment of cancer, comprising a modulator of β-catenin as defined in the invention, which is CKI-7. As long as β-catenin is stabilized, said modulator may be an enhancer or an inhibitor of β-catenin phosphorylation.

It is to be understood that by stabilized β-catenin it is meant such form of β-catenin that does not undergo degradation.

It is disclosed that said pharmaceutical composition is for use in the treatment of cancerous cells of an organism, particularly a human, wherein β-catenin is stabilized. In certain cases where β-catenin is stabilized, and its phosphorylation pathway is impaired, as for example in cancerous cells from colorectal carcinoma or adenoma, the preferred pharmaceutical composition to be used shall comprise an enhancer of β-catenin phosphorylation as the active agent.

Alternatively, wherein β-catenin is stabilized, but its phosphorylation pathway is not impaired, as for example in cancerous cells from malignant melanoma, the pharmaceutical composition to be used shall comprise an inhibitor of β-catenin phosphorylation as the active agent.

The pharmaceutical composition of the invention may further contain additional active agents and/or pharmaceutically acceptable additives, diluents and/or excipients.

The preparation of pharmaceutical compositions is well known in the art and has been described in many articles and textbooks, see e. g. , Gennaro A. R. ed. (1990) Remington's Pharmaceutical Sciences, Mack Publishing Company, Easton, Pennsylvania, and especially pages 1521-1712 therein.

Mutations in β-catenin that affect its phosphorylation have been found in a wide variety of human cancers. The vast majority of colorectal tumors contain mutations in the APC gene, whose product has been shown to be involved in β-catenin phosphorylation. Colorectal tumors lacking an APC mutation are very likely to have a mutation in the β-catenin gene, affecting phosphorylation sites. Interestingly, most of β-catenin mutations found in human cancers are located between seine 29 (S29) and lysine 49 (K49), a region rich in phosphorylation sites and which is also involved in β-catenin degradation. Besides colorectal tumors, such mutations have also been found in tumors such as desmoid (also known as aggressive fibromatosis), endometrial, gastric, hepatocellular, hepatoblastoma, Wilm's tumor (pediatric kidney cancer), medulloblastoma; melanoma, ovarian, pancreatic, pilomatricoma, prostate cancer, thyroid and uterine endometrium tumors [Polakis (2000) *id ibid.*]*.* Thus, stabilization of β-catenin can promote cancer in many tissue types.

The strong correlation between a defective β-catenin phosphorylation pathway and tumor formation (and progression), has made this pathway a focal point for therapy. Restoring β-catenin phosphorylation should halt the cancerous process. There are indications that induced destabilization of β-catenin triggers the death of cancerous cells [Verma et al. (2003) Clin. Cancer Res. 9:1291-300; Kim et al. (2002) Mol Cancer Ther. 1:1355-9]. Axin expression leads concomitantly to β-catenin destruction and apoptosis in tumor cells [Satoh *et al.* (2000) *id ibid*]. Moreover, the inventors' results show that a major effect of axin is to induce S45 phosphorylation and subsequently β-catenin degradation. Thus, the idea that tumor apoptosis is a consequence of the phosphorylation effect of axin is corroborated.

Hence, the results presented herein, showing that S45 phosphoicylation is the priming step for β-catenin phosphorylation, and that modulation of this step can affect β-catenin phosphorylation, are highly applicable for cancer therapy. As shown in Fig. 7, phosphatase -inhibitors, in particular okadaic acid, are important enhancers of 0-catenin phosphorylation.

However, in cancer cells wherein phosphorylated β-catenin is found, it is important to utilize a different modulator. In this case the modulator should be an inhibitor of β-catenin phosphorylation, like CKI-7 for example.

So, in another aspect, the present invention comprises the use of CKI-7 for the treatment of cancer or cancerous cells.

The use of the modulator of the invention is for the treatment of cancerous cells wherein phosphorylation of β-catenin is not impaired. In said cancer (or cancerous cells), said modulator is an inhibitor of β-catenin phosphorylation, which is CKI-7.

The modulator of β-catenin phosphorylation, as disclosed, is to be used in the preparation of a pharmaceutical composition for the treatment of cancer. Alternatively, said pharmaceutical composition is for the treatment of cancerous cells.

As it is demonstrated by the following examples, the fine regulation of β-catenin phosphorylation is critical for cellular homeostasis and it differs in different cell types. In hematopoietic lineages, for example, Wnt can induce the proliferation of multi-potent hematopoietic stem cells through β-catenin stabilization [X. He (2003) Juan March meeting review, Dev. Cell. (In Press)]. When stabilized β-catenin is present in cells (i. e., when it is present in high levels in its non-degradable form) - and said cells are cancerous cells - there are two paths to be followed. Path A, if said β-catenin is non-phosphorylated, as in colon adenomas or carcinomas for example, it is desirable to enhance its phosphorylation in order to induce its degradation. Thus, an enhancer of the invention shall be used to treat said cancer, or cancerous cells. Path B, when said β-catenin is phosphorylated, as in malignant melanomas for example, it is desirable to inhibit its phosphorylation in order to block the transcriptional activation of downstream targets that are potential oncogenes. Thus, an inhibitor of the invention, which is CKI-7, shall be used to treat said cancer, or cancerous cells.

Finally, the disclosure intends to show a method for the treatment of cancer in a subject in need, comprising administering a therapeutically effective amount of the modulator as disclosed, which shall be an enhancer or an inhibitor, according to the specific type of cancer, as specified above.

It must be noted that, as used in this specification and the appended claims, the singular forms "a", "an" and "the" include plural referents unless the content clearly dictates otherwise.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

The following Examples 1-4 and 7 are representative of techniques employed by the inventors in carrying out aspects of the present invention. It should be appreciated that while these techniques are exemplary of preferred embodiments for the practice of the invention, those of skill in the art, in light of the present disclosure, will recognize that numerous modifications can be made without departing from the spirit and intended scope of the invention.

### Examples

### Experimental Procedures

### Western Blot

Western Blots were carried out in the classical method described in 'Molecular Cloning - A laboratory manual by Sambrock et al., Cold Spring Harbor Laboratory Press, 2nd edition, 1989'.

### β-catenin expression system

293T cells were transiently transfected using the calcium phosphate procedure. The following expression vectors were used: Myc-or Flag- tagged β-catenin (0. 5µg) (a human β-catenin clone obtained from R. Grosschedl), Flag-or Myc-tagged axin (2 µg) (mouse cDNA provided by F. Costantini), HA-GSK3β or Flag-GSK3β (3.5 µg) (a rabbit clone obtained from JR Woodgett) [Woodgett, J. R. (1990) EMBO J 9: 2431-2438]. β-catenin mutants include DP, (S33, S37, T40, S45, S47 substituted to alanine) single point mutants (S29F, S33Y, S37A, T41A, S45F) and 45PKA (T41S, T42R, A43R). Additional vectors: Flag-WD, a dominant negative β-TrCP fragment (ΔF-box) [Yaron, A. et al. (1998) Nature 396: 590-594]; Flag-Dvl-1 (a mouse cDlVA clone obtained from S-I Yanagawa) [Lee, J.S. et al. (1999) J Biol Chem 274: 21464-21470]; WT and dominant negative *Xenopus* CKIε (XCKIε-D128N) (provided by T. Shcwarz-Romond) [McKay, R.M. et al. (2001) Dev Biol 235: 378-387]. 24-48 hr after transfection, cells were harvested and processed for the various experiments. MG-132 (Sigma) was used at 20µM for 5 hr; LiCl (40mM) was added for 6 hr, and CKI-7 (100µM) for 16 hr prior to harvesting. β-catenin, axin or GSK-3β in cell lysates were detected using anti-Myc (Ab-1, Oncogenes Research Products, 2µg/ml), anti-Flag (M2, Sigma, 1µg/ml) and anti-HA (12CA5 ascites fluid; 1:5000) antibodies, respectively. GFP expression was monitored with anti-GFP antibody (JL-8, Clontech, 1µg/ml). In some experiments (Fig. 4) GSK-3β was detected using anti-GSK-3β monoclonal antibodies (clone 7, Transduction Laboratories, 0.1 µg/ml). Endogenous and exogenous β-catenin (Figs. 5d and 6a) were detected with anti-β-catenin antibodies (clone 14, Transduction Laboratories) (0.25µg/ml).

### In vitro kinase assays

250 µg protein lysate from Flag-β-catenin 293 transfectants were immunoadsorbed by M2 Flag-affinity beads, and used as a substrate for kinase reactions. Immunobeads were incubated in kinase buffer containing 50mM Tris (pH 7.5), 10 mM MgCl, 5 mM DTT, 5% glycerol, ATP (30µM) and phosphatase inhibitors. Recombinant CKI-δ (aa1-318 fragment, 200U, New England Biolabs), GSK-3β (20U, New England Biolabs) or imniunopurified Flag-axin (0.2 µg protein, peptide-eluted from an immunobead-adsorbed 293 lysate), were added to the reaction mix for 30 min in 30°C. Sequential β-catenin phosphorylation was performed by adding GSK-3β 15 min after CKI-δ and further incubation for 15 min.

### β-catenin phosphorylation analysis

Western blot and mass spectrometry (MS). Three different commercial anti-β-catenin phosplio-peptide antibodies were used: i) Anti-phospho-Thr41/Ser 45 (Cell Signaling Technology), a polyclonal antibody specific for both pT41 and pS45 (αp41,45). ii) Anti-phospho-Ser33/37/Thr41 (Cell Signaling Technology), a polyclonal antibody recognizing pS33 (αp33). These two polyclonal antibodies were used at a 1:1000 dilution, according to the manufacturer's instructions. iii) anti-phospho-Ser 33/37 (BC-22, Sigma), a monoclonal antibody specific for pS37 (used as ascites fluid at a 1:3000 dilution; αp37). Antibody specificities were determined by phosphopeptide inhibition studies. D³²S(PO4)GIHSGATTTAPS⁴⁵ abolished the αp33, but not the αp37 signal; D³²SGIHS(PO4)GATTTAPS⁴⁵ blocked the αp37 but not the αp33 signal. The β-catenin phosphorylation signal of αp41,45 was inhibited by two β-catenin phosphopeptides: partially by G³⁴IHS(PO4)GATT(PO4)TA⁴³ and completely by G³⁸ATT(PO4)TAPS(PO4)LS⁴⁷, indicating that both pT41 and pS45 are recognized by the antibodies. Proteins for MS analysis were immunopurified by M2 Flag-affinity beads (Sigma), separated by SDS-PAGE, Coomassie stained and the β-catenin bands were in-gel digested with endoproteinase Asp-N. The resulting peptides were desalted on small columns, eluted with 20% MeOH, 5% HCOOH, and analyzed by nanoelectrospray mass spectrometry [Yaron *et al*. (1998) *id ibid*.], using a quadrupole time-of-flight mass spectrometer (PE-Sciex, Canada).

### Momtoring β-catenin transcriptional transactivation

Cells were transfected with the TOPFLASH or pGL3-MITF/M luciferase reporter plasmid [Korinek et al. (1997) Science, 275: 1752-3; Takeda et al. (2000) J. Biol. Chem. 275:14013-6] and the relevant expression plasmid combinations. 24 hours after transfection the cells were harvested. Luciferase activity was determined in the cells' lysate using Promega's Luciferase Assay System. The transcription activity is expressed in Relative Luciferase Units (RLU). TCF mutant-site reporters (FOPFLASH and pGL3-MITF/M195.3) were used to control TCF-independent transcriptional transactivation.

### Example 1

### Axin induces β-catenin phosphorylation-degradation cascade, initiated by phosphorylation at serine residue 45.

To study the phosphorylation cascade that promotes β-catenin degradation, a simple protein expression system was set up in 293 cells: the combined overexpression of axin and GSK-3β triggers the degradation of exogenously-expressed β-catenin (Myc-tagged) (Fig. 1a). In this system, neither component alone was sufficient to promote β-catenin degradation over a wide range of plasmid expression (Fig. 1a and data not shown). Degradation was blocked upon cell treatment with the proteasome inhibitor MG-132 (Fig. 1b) or by overexpression of a dominant negative β-TrCP, a recognized β-catenin ubiquitin ligase (E3) [Polakis (2000) *id ibid*.] (Fig. 1a, lane 7). Cell lysates were analyzed for β-catenin phosphorylation using a series of commercial anti-β-catenin phosphopeptide antibodies: antibody specific for both pT41 and pS45 (αp41,45), and antibodies recognizing either pS33 or pS37. No phosphorylation was detected upon transfection of Myc-β-catenin alone, or in combination with GSK-3β (Fig. 1b, lanes 2, 3). Axin expression induced a prominent signal with αp41,45 and minor signals with αp33 and αp37, possibly attributable to endogenous GSK-3β activity (lane 5). Coexpression of axin and GSK-3β enhanced S33 and S37 phosphorylation to a great extent (10-30 fold), whereas the T41/S45 pliosphorylation signal was modestly enhanced (2-4 fold, compare lanes 4, 5), probably due to GSK-3β-mediated T41 phosphorylation. In contrast, an N-terminal mutated β-catenin (dominant positive, DP) did not show any phosphorylation signal and was stable, irrespective of proteasomal inhibition (Fig. 1a, b). S45 phosphorylation was also observed upon overexpression of APC, yet to a much lower extent (data not shown). This may suggest that APC has a role in the priming reaction, which is likely limited to supporting the activity of axin. Indeed, mutational analysis of APC in cancer, has shown that many APC mutations prohibit its engagement with axin, while preserving β-catenin association [Hart, M.J. et al. (1998) Curr Biol 8: 573-581; Fearnhead, N.S. et al. (2001) Hum Mol Genet 10: 721-733].

To resolve the phosphorylation specificity of axin and GSK-3β, β-catenin phosphorylation was further analyzed by mass spectrometry (MS). MS analysis showed trace phosphorylation of the N-terminal region of β-catenin when transfected alone (Fig. 1c, panels 1, 2). Axin coexpression resulted in a major phosphorylation signal at S45, with barely detectable phosphorylation signals at any other potential N-terminal phosphorylation site (panels 3, 4, 7). Conversely, the combined expression of axin and GSK-3β resulted in phosphorylation signals at S45 and four consecutive N-terminal phosphorylation sites: T41, S37, S33 (panels 6, 8) and S29 (not shown), with decreasing signal intensity.

β-catenin studies in a variety of human tumors indicated that several potential N-terminal phosphorylation sites are often mutated, leading to stabilization and enhanced nuclear expression of the mutated protein [Morin *et al*. (1997) *id ibid.;* Rubinfeld *et al*. (1997) *id ibid.;* Wong *et al.* (2001) *id ibid.].* Many of these tumor mutations are concentrated around the consensus binding site of β-TrCP [Yaron *et al*. (1998) *id ibid.*] (DS*GXXS*, S* denotes phosphoserine), accounting for β-catenin stabilization. However, two common mutation sites, S45 and T41, are positioned C-terminally to the canonical β-TrCP recognition motif. Thus, stabilization of β-catenin by T41/S45 mutations calls for a different explanation. One possibility is the formation of a redundant E3 anchoring site around pT41 and pS45 (S*XXXS*) [Aberle *et al*. (1997) *id ibid;* Yaron *et al*. (1998) *id ibid.*]*,* which is absent in the mutants. The other possibility is that these mutations influence the phosphorylation of S33 and S37, which is necessary for generating the β-TrCP binding site. To address this issue, a series of point mutations at the MS-detected N-terminal phosphorylation sites were created and their phosphorylation and degradation were examined in the 293 system. Expression of axin or axin-GSK-3β yielded phosphorylation signals with αp41,45 in all the mutants, aside from S45F (Fig. 2a). This pertains also to the T41A mutant, which can only be phosphorylated on S45, indicating that axin may induce an exclusive S45 phosphorylation. On the other hand, none of the mutants, with the exception of S29F, was phosphorylated both at S33 and S37 in response to axin-GSK-3β transfection (Fig. 2a). Consistent with their phosphorylation status, only wt and S29F β-catenin were degraded following co-expression of axin-GSK-3β (Fig. 2b). All the other mutants resisted degradation, in accordance with the stability of their tumor counterparts in human cancer [Polakis (2000) *id ibid.].* As mutations at S37 and S33 allow T41/S45 phosphorylation (Fig. 2a, lanes 7-12), but resist degradation (Fig. 2b, lanes 5-8), the occurrence of a functionally-redundant β-TrCP recognition site downstream of S37 is unlikely. Therefore, it appears that phosphorylation at S45 initiates a linear GSK-3β cascade, in which each phosphorylation site serves as a necessary priming site for the successive one. Although it runs all the way through S29, the aim of the cascade is apparently restricted to generating the canonical β-TrCP recognition site around S33/37. This presumption is supported by the prevalence of tumor mutations at Asp32 and Gly34 [Wong *et al*. (2001) *id ibid*.], which do not affect the phosphorylation, but are likely to compromise the β-TrCP recognition motif.

### Example 2

### S45 phosphorylation, which by itself is GSK-3β-independent, is both necessary and sufficient to initiate a GSK-3β-dependent phosphorylation-degradation cascade

The above experiments implicate axin in S45 phosphorylation, but do not rule out contribution of GSK-3β to this event. GSK-3β is traditionally known to target the phosphorylation of +4P-primed substrates [Frame, S. et al. (2001) Mol Cell 7: 1321-1327], a specificity supported by recent structural studies of the enzyme [Dajani, R. et al. (2001) Cell 105: 721-732]. The fact that the S45 phosphorylation site is not preceded by a +4P priming site led to the proposition that the molecular complex of axin and GSK-3β is capable of bypassing the priming requirement of the uncomplexed enzyme [Cohen and Frame (2001) *id ibid.].* To assess the contribution of GSK-3β in axin-mediated S45 phosphorylation, two types of experiments were carried out. In the first set, 293 cells were incubated prior to harvesting with LiCl, a GSK-3β inhibitor capable of mimicking a Wnt effect [Klein, P.S. and D.A. Melton (1996) Proc Natl Acad Sci U S A 93: 8455-8459; Shambolic, V. et al. (1996) Curr Biol 6: 1664-1668]. Whereas the modest axin-induced S33/37 phosphorylation of wt β-catenin was abolished by LiCl, the counterpart T41/S45 phosphorylation signal was minimally affected (Fig. 3a, compare lanes 3, 6). Similarly, no difference was observed in the MY analysis of S45 peptides from LiCl-treated versus non-treated cells (data not shown). Moreover, LiCl treatment had no effect on S45 phosphorylation of a T41A mutated β-catenin (Fig. 3a, compare lanes 3, 6), indicating that S45 phosphorylation was independent of GSK-3β.

In the second set of experiments, a mutated axin (Leu 525 converted to Pro, [L525P-axin]), which is incapable of interacting with GSK-3β was tested (Fig. 3b) [Smaller, M.J. et al. (1999) EMBO J 18: 2823-2835; Rubinfeld, B. et al. (2001) J Biol Chem 276: 39037-39045]. L525P-axin was as effective as wt axin in inducing S45 Phosphorylation (Fig. 3c, lanes 2,4) and MS analysis [see below, Fig. 5c]). Unlike wt axin, the L525P mutant is unable to engage the endogenous GSK-3β for phosphorylating S33/37 (Fig. 3c, lanes 2 vs. 4). However when complimented by exogenous GSK-3β, L525P and wt axin generated a comparable S33/37 phosphorylation signals (Fig. 3c, lanes 3, 5, and MS data [not shown]) and β-catenin degradation (Fig. 3c lane 7 and Fig. 1a, lane 6). Cumulatively, these experiments show that the priming phosphorylation at S45 does not require GSK-3β, but a different protein kinase.

These data show that S45 phosphorylation is essential for initiating GSK-3β phosphorylation, yet do not indicate whether it is sufficient to mobilize the cascade. To address this question, the inventors constructed a β-catenin containing surrogate protein kinase A (PKA)-mediated phosphorylation site at S45 (45PKA). This manipulation did not affect the expression or stability of β-catenin (data not shown), but resulted in its constitutive S45 phosphorylation in 293 cells (Fig. 4, lane 1), which was inhibited by H89, a specific PKA inhibitor (data not shown). S45 phosphorylation of 45PKA did not require axin, nor was it enhanced by axin overexpression (Fig. 4, lanes 1 and 2). Therefore, 45PKA proved instrumental in studying the initiation and progression of the GSK-3β phosphorylation cascade independently of axin. GSK-3β transfection resulted in pronounced S33/37 phosphorylation of the 45PKA mutant (lane 3), as well as its complete degradation (lane 5). This is in striking contract to the inability of GSK-3β to induce phosphorylation and degradation of wt β-catenin when transfected alone (Fig. 1). To control the effect of wt GSK-3β, an R96A-GSK-3β mutant was used, which cannot accommodate a priming phospho-serine and is therefore, unable to phosphorylate priming-dependent substrates [Frame *et al.* (2001) *id ibid.*]. R96A-GSK-3β did not induce 45PKA phosphorylation at S33/37 (Fig. 4, lane 4) and its subsequent degradation (lane 6). These results indicate that β-catenin is a *bona fide* priming-dependent substrate for GSK-3β and that S45 priming is both necessary and sufficient for driving the GSK-3β cascade. Nevertheless, the above experiments cannot address additional roles for axin in the cascade. It is possible that when GSK-3β is present in limiting amounts, axin helps to recruit GSK-3β to β-catenin. In support of such a role are the data comparing the effect of wt axin with that of L525P-axin: the latter, which is incapable of associating with endogenous GSK-3β, failed to induce S33/37 phosphorylation (Fig. 3c, lane 4).

### Example 3

### Axin-induced S45 phosphorylation is mediated by CKI

To identify the axin-associated priming kinase, Flag-axin was immunopurified from 293-transfected cells and analyzed its endogenous associated proteins by LC/MS. Only 5 protein kinases were detected in association with axin at a high score: The two GSK-3 isoforms, α and β, and three CKI isoforms, ε, δ and α. These CKI isoforms have a highly conserved kinase domain and appear to have similar or identical substrate specificity [Fish, K.J. et al. (1995) J Biol Chem 270: 14875-14883]. Several studies implicated CKIε in the Wnt pathway, mostly as a positive effector [Peters, J.M. et al. (1999) Nature 401: 345-350; Lee, E. et al. (2001) J Cell Biol 154: 983-993; McKay *et al.* (2001) *id ibid.;* Gao, Z.H. et al. (2002) Proc Natal Acad Sci U S A 99: 1182-1187]. CKIε has been shown to interact with axin [Sakanaka, C. et al. (1999) Proc Natl Acad Sci U S A 96: 12548-12552; Rubinfeld *et al.* (2001) *id ibid.]* and it was recently proposed that this kinase mediates axin-induced APC phosphorylation, thereby stabilizing the β-catenin degradation complex [Rubinfeld *et al.* (2001) *id ibid.].* Therefore, CKI was evaluated as a candidate S45-kinase in both *in vitro* and *in vivo* assays.

First, the *in vitro* phosphorylation of β-catenin was tested using an immunopurified Flag-axin (the LC/MS preparation) and the recombinant enzymes CKIδ (a N-terminal 318aa fragment) and GSK-3β (Fig. 5a). Both CKIδ and axin induced the phosphorylation of β-catenin at S45/T41, but not at S33 (lanes 3,7). To directly implicate CKI in priming the GSK-3β-phosphorylation cascade, β-catenin was subjected to sequential phosphorylation by CKI followed by GSK-3β. GSK-3β was incapable of phosphorylating β-catenin on its own (lane 4), yet induced a pronounced pS33 signal following S45 phosphorylation by CKI (lane 5).

In another set of experiments the *in vivo* role of CKI in S45 phosphorylation was analyzed, using two dominant negative CKIε constructs (dnXCKIs K85R and D128N) [McKay *et al*. (2001) *id ibid*.] and a specific CKI inhibitor (CKI-7) [Chijiwa, T. et al. (1989) J Biol Chem 264: 4924-4927]. Coexpression of the two dnXCKIε, but not the wt kinase, with axin, suppressed the ability of axin to induce S45 phosphorylation in 293 cells (Fig. 5b, lanes 5,4,3). Next, the effect of CKI-7 on axin-induced S45 phosphorylation was tested, in comparison to its effect on the constitutive phosphorylation of 45PKA. CKI-7 treatment diminished the axin-induced p45 signal of wt β-catenin, but did not affect the p45 phosphorylation of 45PKA (Fig. 5c). The inhibitor effect was further tested on S45 phosphorylation of endogenous β-catenin in proteosome-inhibited HeLa cells (Fig. 5d): CKI-7 suppressed both S45/T41 and S83 phosphorylation (lane 3), while the GSK-3β inhibitor LiCl exclusively blocked S33 phosphorylation (lane 6). In the same experiment, H89, a PKA inhibitor, had no effect on wt β-catenin phosphorylation (lane 5). Cumulatively, these *in vitro* and *in vivo* results indicate that CKI mediates the function of axin in initiating the β-catenin phosphorylation-degradation cascade.

The LC-MS/MS analysis of the S45 phosphorylation complex revealed three CKI isoforms in association with immunopurified axin. To determine which CKI was responsible for S45 phosphorylation, RKO cells were treated with siRNA oligonucleotides of CKIα, or a common siRNA for CKIε and CKIδ. β-catenin phosphorylation on S45 and p33 was determined 72 hours after siRNA treatment, in comparison to treatment with proteasome inhibitors alone (Fig 5e). Western blot analysis shows a reduced expression of the CKIα and CKIε, along with a remarkable reduction of the phosphorylation signals at both S45 and S33. Hence it appears that the two CKI isoforms have a comparable effect in inducing S45 phosphorylation in RKO cells.

### Example 4

### S45 phosphorylation is regulated by Wnt signaling through Dvl

The control of β-catenin stability is a major task of the Wnt pathway and is mediated through members of the conserved protein family dishevelled (Dvl 1,2,3) [Boutros and Mlodzik (1999) *id ibid.*]*.* The current model suggests that Dvl relays the Wnt signal while associated with axin [Kishida, S. et al. (1999) Mol Cell Biol 19: 4414-4422; Smalley *et al*. (1999) *id ibid.;* Salic, A. et al. (2000) Mol Cell 5: 523-532]. As the previous results showed that axin controls the initiating event in the β-catenin phosphorylation-degradation cascade, it remained to be seen whether Wnt and Dvl regulate S45 phosphorylation. To this end, several cell lines were first stimulated with Wnt3A [Roelink, H. and R. Nusse. (1991) Genes Dev 5: 381-388; Shibamoto, S. *et al*. (1998) *id ibid.*; Lee *et al*. (1999) *id ibid.*], and S45 phosphorylation was monitored by Western blot analysis. Wnt stimulation stabilized endogenous β-catenin in mouse L-cells (fibroblasts) (Fig. 6a, lanes 1,2) and exogenous β-catenin in Jurkat lymphocytes (lanes 5,6). It did not affect the levels of an already stabilized S37 mutated β-catenin in the SNU449 hepatoma cell line [Satoh, S. et al. (2000) Nat Genet 24: 245-250] (lanes 9-11). S45 phosphorylation of β-catenin was evident in proteasome-inhibited L cells (lane 3), Jurkat cells (lane 7) and HeLa cells (lane 14). It was also apparent in okadaic acid-treated SNU449 cells (OKA, lane 10). Wnt3A treatment resulted in inhibition of S45 phosphorylation in all four cell lines (lanes 4, 8, 11, 15), and the subsequent inhibition of S33/37 phosphorylation in Jurkat, HeLa and L cells (lanes 8, 15, 4), suggesting that Wnt signaling intervenes between axin and S45 phosphorylation.

To examine the role of Dvl in the regulation of S45 phosphorylation, mouse Dvl-1 [Lee *et al*. (1999) *idi ibid*.] was introduced into the 293 system and S45 phosphorylation was evaluated by Western blot and MS analysis. Dvl transfection resulted in nearly complete inhibition of axin-induced S45 phosphorylation (Fig. 6c, lanes 5, 6), in parallel to stabilizing β-catenin (lane 9). A similar effect of Dvl was observed for the L525P-axin-induced S45 phosphorylation. MS (Fig. 6b) and Western blot analysis (data not shown) indicated that Dvl inhibits the axin-CKI-induced S45 phosphorylation. However, Dvl, as a Wnt signal mediator, could also regulate the subsequent steps of the GSK-3β phosphorylation cascade. To evaluate this possible dual role of Dvl, Dvl was co-expressed with the 45PKA β-catenin mutant that initiates the GSK-3β cascade independently of axin (Fig. 4). Dvl had no effect on PKA-mediated S45 phosphorylation (Fig. 6d, compare lanes 1, 2), nor did it block the GSK-3β-mediated S33/37 phosphorylation (Fig. 6d, lanes 3, 4). Likewise, in striking difference to Dvl's stabilizing effect on wt β-catenin (Fig. 6c, lane 9), 45PKA degradation was not inhibited by Dvl (Fig. 6d, lanes 6, 7). This finding indicates that the axin/CKI-mediated S45 phosphorylation event, rather than the resulting GSK-3β phosphorylation-cascade, is the critical target for Wnt signaling.

### Example 5

### Induction of pSer45 and pSer33 phosphorylation by Okadaic acid

Okadaic acid is a selective inhibitor of phosphatase PP2A, while it inhibits to a lower extent (10-50 fold less) phosphatase PP1. Proteasome-inhibited HeLa and SW480 cells (treated with 20 µM MG132) or Ser 37-mutated SNU 449 colon carcinoma cells [Satoh *et al*. (2000) *id ibid.*] were treated with okadaic acid (1 µM) for 1 or 3hr, harvested and their lysate analyzed by Western blot with anti-phospho β-catenin antibodies: anti-pSer45 and anti-pSer33 (for HeLa and RKO cells only). As seen in Fig. 7, okadaic acid treatment induces a massive phosphorylation signal with both antibodies. Yet, since S45 phosphorylation precedes the S33 phosphorylation, the okadaic effect may be reserved to S45. This is an example of a compound that can upregulate S45 phosphorylation, while Wnt3A downregulates the same phosphorylation event (Fig. 6a).

### Example 6

### FRAT1 promotes β-catenin phosphorylation

Overexpression of Frat1 in 293 cells together with axin and GSK3 resulted in hyper-phosphorylation of β-catenin on the S45 GSK3-priming (not shown) and the successive GSK3 phosphorylation sites (T41, S37 and S33, Fig. 9a). Surprisingly phosphorylated β-catenin was not degraded, despite the absence of proteasome inhibitors, and instead, accumulated in the trasfected cells (Fig. 9a). Co-expression of low amounts of Dvl in these cells did not repress S45 and S33 phosphorylation, but on the contrary, augmented the phospho-β-catenin stabilization effect of Frat1 (Fig. 9a). Higher expression of Dvl suppressed β-catenin phosphorylation, probably due to its predominant effect on S45 phosphorylation (Fig. 9a). Unlike the wt Dvl effect, 293 transfection with a Dvl deletion mutant devoid of the Frat-interaction motif, PDZ (Dvl-ΔPDZ), resulted in stabilization of non-phosphorylated β-catenin (Fig. 9b). Dvl-ΔPDZ suppresses S45 phosphorylation, yet cannot activate Frat and thus is incapable of stabilizing phosphorylated β-catenin:

293 cells were transfected with the TOPFLASH luciferase reporter plasmid and the expression plasmids for β-catenin, axin, GSK, Frat and Dvl, according to the combinations indicated in Figure 9c. 24 hours after transfection, the cells were harvested and their lysate luciferase activity was determined using Promega's Luciferase Assay System. TCF reporter assays indicated that the robustly phosphorylated β-catenin obtained upon Frat1 overexpression in 293 cells functions as an effective TCF/LEF co-activator, far better than Dvl-stabilized β-catenin (Fig. 9c).

p300/CBP is a transcriptional activator, which has been noted to cooperate with β-catenin in the activation of TCF-regulated genes [Hecht, A. et al. (2000) EMBO J. 19(8): 1839-50]. 293 cells were transfected with the expression plasmids for Myc-β-catenin, p300/CBP, E1A and Frat, as indicated in Figure 9d. p300/CBP and E1A stabilize phospho-β-catenin similarly to Frat1.

In some cases the transcriptional effects of p300/CBP are mediated via its acetyl-transferase activity [Chan, H. M. and La Thangue, N. B. (2001) J. Cell Sci. 114(Pt13):2363-73]. Interestingly, overexpression of both p300/CBP and Frat1 was found to induce lysine-acetylation of β-catenin (Fig. 9e). Acetylated β-catenin species concur with the phosphorylated ones, indicating a possible linkage of the two events. Protein acetylation prohibits the ubiquitination of the modified lysine residues and thus may result in the stabilization of a modified protein [Brooks and Gu (2003) Curr. Opin. Cell Biol. 15(2): 164-71]. E1A may function both as a negative and positive regulator of p300/CBP acetyl-transferase activity [Chan and La Thangue (2001) *id ibid.;* Gallimore and Turnell (2001) Oncogene 20(54): 7824-35]. At lower concentrations, E1A functions to recruit p300/CBP, and thereby can facilitate protein-acetylation [Ait-Si-Ali, S. et al. (1998) Nature 3 96(6707):184-6], whereas at high concentrations it antagonizes the acetyl-transferase activity of p300/CBP [Li, Q. et al. (1999) EMBO J. 18(20): 5634-52]. This may explain the opposing effects of E1A in combination with p300/CBP and Frat1 on β-catenin phosphorylation: whereas low E1A concentrations enhance the stabilization of phosphorylated β-catenin, higher E1A concentrations suppress the accumulation of phosphorylated β-catenin ( Fig. 9d). The inventor's experiments therefore indicate that certain oncogenes such as Frat1 and E1A have the capacity to induce the stabilization of phosphorylated β-catenin, possibly via protein acetylation.

### Example 7

### CKI inhibition suppresses β-catenin phosphorylation

MITF (*Microphtalmia*-associated transcription factor) is a lineage-determination transcription factor, which modulates melanocyte differentiation and pigmentation, functioning downstream of the Wnt pathway. MITF has been used as a marker of melanoma cells, and has been shown to be required for β-catenin induction of melanoma growth [Widlund *et al.* (2002) *id ibid*.]*.*

RKO cells were transfected with the pGL3-MITF/M luciferase reporter plasmid [Takeda *et al.* (2000) *id ibid*.] and the expression plasmid combinations indicated in the graph (Fig. 10a). 24 hours after transfection the cells were harvested and their lysate luciferase activity was determined using Promega's Luciferase Assay System. Co-transfection of an MITF reporter plasmid with Frat1 into RKO cells augments its transcription (Fig. 10a), whereas dominant-negative CKI (DN-CKI) plasmids were found to reduce both the basal and Frat1-enhanced MITF' reporter activity. Dominant-negative CKIs function by suppressing S45 phosphorylation of β-catenin, causing β-catenin accumulation in the cells in its non-phosphorylated form (see Fig. 5b). Since non-phosphorylated β-catenin is not affected by Frat1, β-catenin did not accumulate in the Frat1-transfected RKO cells and consequently, there was no transcription from the MITF reporter (Fig. 10a).

The highly metastatic melanoma cell line LB33B1 (B1) is a derivative of the low grade malignant LB33A1 (A1) cells [Ikeda H. et al. (1997) Immunity 6(2): 199-208]. Both cell lines harbor a similar high content of β-catenin, irrespective of proteasome inhibition, yet the B1 β-catenin is phosphorylated (Fig. 10b). The inventors show that the MITF reporter is highly active in the B1 cells, but not in the A1 melanoma (Fig. 10c). To assess the contribution of phosphorylated versus non-phosphorylated β-catenin to the MITF-transcriptional activity, reporter-transfected B1 cells were treated with LiCl, which suppresses GSK3 phosphorylation, or cotrasfected with Dvl-APDZ, which cannot cooperate with Frat1, yet suppresses S45 phosphorylation. Both the LiCl treatment and Dvl-ΔPDZ transfection resulted in the inhibition of the MITF promoter activity (Fig. 10c) despite the presence of stable β-catenin (Fig. 10b). Abrogating β-catenin phosphorylation in melanoma cells may compromise their growth and indeed, treatment of B1 melanoma cells with CKI7, a small-molecule specific CKI inhibitor, slowed their proliferation (Fig. 10d) along with suppressing the phosphorylation of β-catenin (data not shown). Thus, the inventors' experiments suggest that S45-phosphorylation inhibitors, such as CKI7 may constitute an effective means of chemotherapy of certain malignant diseases (e.g., malignant melanoma).

## Claims

1. A method of screening for an anti-cancer agent, wherein said method comprises the steps of:
(a) contacting an agent with a reaction mixture comprising β-catenin, axin and/or casein kinase I alpha (CKla),
(b) incubating said mixture under conditions which allow a change in phosphorylation of β-catenin at serine 45 (S45), and
(c) detecting phosphorylation of β-catenin at S45,
whereby enhanced phosphorylation of β-catenin at S45 indicates that said agent is useful as an anti-cancer agent.

2. Use of CKI-7 for the manufacture of a medicament identified for the treatment of cancer.

3. The use of claim 2, wherein β-catenin of said cancer is stabilized and its phosphorylation is not impaired.

4. The use of claim 3, wherein said cancer is malignant melanoma.

5. A pharmaceutical composition comprising CKI-7.

6. Use of a siRNA of CKI-α for the manufacture of a medicament identified for the treatment of cancer.

7. A pharmaceutical composition comprising a siRNA of CKI-α.

## Patentansprüche

1. Verfahren zum Screening eines Antikrebsmittels, wobei das Verfahren die Schritte umfasst:
(a) Kontaktieren eines Mittels mit einer Reaktionsmischung umfassend β-Catenin, Axin und/oder Casein Kinase I alpha (CKIα),
(b) Inkubieren der Mischung unter Bedingungen, die eine Veränderung der Phosphorylierung von β-Catenin an Serin 45 (S45) ermöglichen, und
(c) Detektieren der Phosphorylierung von β-catenin an S45,
wobei eine erhöhte Phosphorylierung von β-Catenin an S45 anzeigt, dass das Mittel als ein Antikrebsmittel nützlich ist.

2. Verwendung von CKI-7 zur Herstellung eines Medikaments, das für die Behandlung von Krebs bestimmt ist.

3. Verwendung nach Anspruch 2, wobei β-Catenin aus dem Krebs stabilisiert ist und seine Phosphorylierung nicht beeinträchtigt ist.

4. Verwendung nach Anspruch 3, wobei der Krebs malignes Melanom ist.

5. Pharmazeutische Zusammensetzung umfassend CKI-7.

6. Verwendung einer siRNA von CKI-α zur Herstellung eines Medikaments, das für die Behandlung von Krebs bestimmt ist.

7. Pharmazeutische Zusammensetzung umfassend eine siRNA von CKI-α.

## Revendications

1. Procédé de criblage pour un agent anticancéreux, où ledit procédé comprend les étapes de :
(a) mise en contact d'un agent avec un mélange réactionnel comprenant de la β-caténine, de l'axine et/ou de la caséine kinase 1 alpha (CKIα),
(b) incubation dudit mélange dans des conditions qui permettent un changement dans la phosphorylation de la β-caténine à la sérine 45 (S45), et
(c) détection de la phosphorylation de la β-caténine à S45,
où la phosphorylation accrue de la β-caténine à S45 indique que ledit agent est utile comme agent anticancéreux.

2. Utilisation de CKI-7 pour la fabrication d'un médicament identifié pour le traitement du cancer.

3. Utilisation selon la revendication 2 où la β-caténine dudit cancer est stabilisée et sa phosphorylation n'est pas dégradée.

4. Utilisation selon la revendication 3 où ledit cancer est le mélanome malin.

5. Composition pharmaceutique comprenant CKI-7.

6. Utilisation d'un siRNA de CKI-α pour la fabrication d'un médicament identifié pour le traitement du cancer.

7. Composition pharmaceutique comprenant un siRNA de CKI-a.
